# EUROPEAN PATENT APPLICATION

(11) **EP 4 410 254 A1**
(43) Date of publication of application: **07.08.2024**
(21) Application number: 22883472.7
(22) Date of filing: 13.10.2022
(51) Int. Cl.: A61F 13/15

(54) **METHOD FOR MANUFACTURING ABSORBENT ARTICLE**

(30) Priority: 18.10.2021 JP 2021170579
(71) Applicant: Zuiko Corporation, Ibaraki-shi Osaka 5670082 (JP)
(72) Inventor: SHIMADA, Takahiro, Ibaraki-shi, Osaka 567-0082 (JP); IKEDA, Natsuki, Ibaraki-shi, Osaka 567-0082 (JP)
(74) Representative: Hasegawa, Kan
(86) International application number: PCT/JP2022/038250
(87) International publication number: WO 2023/068168

(57) **Abstract**

Provided is a method for manufacturing absorbent articles that readily suppresses an area in which a manufacturing device is installed. The method for manufacturing absorbent articles includes a conveying step (S 1), a moving step (S4), and a supplying step (S6). In the conveying step, absorbent articles aligned in multiple rows along a first direction are conveyed on a first conveying path in a second direction orthogonal to the first direction. In the moving step, rows of absorbent articles that are either even-numbered or odd-numbered when counted along the first direction from the row of absorbent articles farthest upstream in the first direction are moved to a second conveying path different from the first conveying path. In the supplying step, after rows of absorbent articles of divided groups have been moved to the second conveying path, the rows of absorbent articles are conveyed in the first conveying path and the second conveying path in a direction orthogonal to a longitudinal direction of the absorbent articles, and are supplied into respective folding devices corresponding to each row of absorbent articles among a plurality of devices for folding the absorbent articles.

## Description

### TECHNICAL FIELD

The present invention relates to a method for manufacturing absorbent articles.

### BACKGROUND ART

Conventionally, manufacturing methods in which absorbent articles are manufactured in multiple rows are known, as in Patent Literature 1 (Japanese Laid-open Patent Publication No. 60-225554).

### PRIOR ART LITERATURE

### <PATENT LITERATURE>

(Patent Literature 1) Japanese Laid-open Patent Publication No. 60-225554

### SUMMARY OF THE INVENTION

### <Technical Problem>

In the manufacturing method disclosed in Patent Literature 1 (Japanese Laid-open Patent Publication No. 60-225554), a process is performed in which sheets are combined with absorbers that are conveyed in multiple rows, whereupon the rows are separated, a width of a conveyance path is expanded, and the absorber/sheet combinations are conveyed. Such a method for manufacturing absorbent articles has a problem in that an installation area of a device for manufacturing absorbent articles increases due to the widening of the conveyance path.

In addition, in the manufacturing method disclosed in Patent Literature 1 (Japanese Laid-open Patent Publication No. 60-225554), after the absorbers and the sheets have been combined, a process is performed to separate the sheets into a plurality of parts in a direction perpendicular to a conveyance direction. In such a manufacturing method, the sheets and the absorbers are disposed adjacent to each other, whereby force acting on the sheets is no longer uniform; therefore, there is a risk that when the sheets are cut, inappropriate force will be applied locally to the sheets and the sheets will be wrinkled or twisted.

In view of these problems, an object of the present invention is to provide a method for manufacturing absorbent articles with which the installation area of the manufacturing device tends to be suppressed.

Another object of the present invention is to provide a method for manufacturing absorbent articles with which wrinkles and twists are less likely to occur in the sheets constituting the absorbent articles.

### <Solution to Problem>

A method for manufacturing absorbent articles according to one aspect of the present invention comprises a conveyance step, a moving step, and a supplying step. In the conveyance step, absorbent articles aligned in multiple rows along a first direction are conveyed on a first conveyance path in a second direction orthogonal to the first direction. In the moving step, rows of absorbent articles that are either even-numbered or odd-numbered when counted along the first direction from the row of absorbent articles farthest upstream in the first direction are moved from a first conveying path to a second conveying path as rows of absorbent articles of divided groups while remaining in the same state of alignment in the first direction. The supplying step is performed after the rows of the absorbent articles of divided groups have been moved to the second conveying path. In the supplying step, the rows of absorbent articles are conveyed in a direction orthogonal to a longitudinal direction of the absorbent articles in the first conveying path and the second conveying path, and are each supplied into a processing device that corresponds to that row of absorbent articles among a plurality devices for processing the absorbent articles.

In a method for manufacturing absorbent articles according to another aspect of the present invention, each absorbent article has an absorber and a sheet piece arranged adjacent to the absorber. The method for manufacturing absorbent articles comprises a forming step, an arrangement step, a joining step, and a cutting-out step. In the forming step, at least a first raw material sheet among conveyed raw material sheets is divided into a plurality in a direction orthogonal to a conveyance direction of the first raw material sheet, and a plurality of divided sheets are formed. In the arrangement step, a plurality of sheet members and absorbers are arranged. The plurality of sheet members include the divided sheets formed from the first raw material sheet, and either a raw material sheet other than the first raw material sheet or divided sheets formed by dividing a raw material sheet other than the first raw material sheet into a plurality in a direction orthogonal to the conveyance direction. In the arrangement step, the absorbers are arranged between at least two sheet members. In the joining step, the absorbers and the corresponding sheet members are joined. In the cutting-out step, the sheet members joined to the absorbers are cut and the sheet pieces are cut out.

### < Effects of Invention>

In the method for manufacturing absorbent articles according to one aspect of the present invention, absorbent articles aligned in multiple rows in a direction orthogonal to the conveyance direction are separated into odd-numbered rows and even-numbered rows, and are then supplied into absorbent-article-processing devices corresponding to respective rows of absorbent articles. Therefore, even if a distance between the rows of absorbent articles is not expanded, space around the rows of absorbent articles is readily ensured when the absorbent articles are supplied into the processing devices. As a result, it is possible to suppress the incidence of events in which absorbent articles in adjacent rows impede absorbent articles from being supplied into the processing device of each row, without expanding the width at which the absorbent articles are conveyed.

In the method for manufacturing absorbent articles according to another aspect of the present invention, the first raw material sheet, which is a raw material sheet, is divided into a plurality in a direction orthogonal to the conveyance direction of the first raw material sheet, and a step of arranging the absorbers and the sheet members including the divided sheets is performed after the plurality of divided sheets have been formed. Therefore, it is possible for the incidence of events in which inappropriate force is applied locally to the first raw material sheet when the first raw material sheet is cut and the sheet members constituting the absorbent articles become wrinkled or twisted, to be suppressed compared to a case where the first raw material sheet is divided into a plurality in a direction orthogonal to the conveyance direction of the first raw material sheet after the absorbers and the first raw material sheet have been arranged adjacent to each other.

### BRIEF DESCRIPTION OF THE DRAWINGS

FIG. 1 is a figure of a pre-folded state of an absorbent article manufactured using the method for manufacturing absorbent articles of the present invention;
FIG. 2 is a partial schematic cross-sectional view taken along arrow II-II of FIG. 1;
FIG. 3 is a diagram of a flow of steps for forming a pre-folding absorbent article from a first sheet, a second sheet, and an absorber;
FIG. 4A depicts a first sheet being conveyed;
FIG. 4B depicts a state in which slits have been formed in the conveyed first sheet along a conveyance direction of the first sheet, the slits being depicted as double-dashed lines;
FIG. 4C depicts a state in which a conveying width of a plurality of first divided sheets, having been divided by the slits, is expanded in a direction orthogonal to the conveyance direction of the first divided sheets so as to increase a distance between adjacent first divided sheets;
FIG. 5A depicts a second sheet being conveyed;
FIG. 5B depicts a state in which slits have been formed in the conveyed second sheet along a conveyance direction of the second sheet, the slits being depicted as double-dashed lines;
FIG. 5C depicts a state in which a conveying width of a plurality of second divided sheets, having been divided by the slits, is expanded in a direction orthogonal to the conveyance direction of the second divided sheets so as to increase a distance between adjacent second divided sheets;
FIG. 6 depicts a conveying state of absorbers;
FIG. 7 depicts a conveying state of integrated sheets in which first divided sheets, second divided sheets, and absorbers are integrated;
FIG. 8 is a cross-sectional view taken along arrow VIII-VIII of FIG. 7;
FIG. 9 shows a state in which multiple rows of absorbent articles formed from the multiple rows of integrated sheets in FIG. 7 are conveyed on a first conveying path;
FIG. 10 is a schematic drawing of a configuration of a manufacturing device that manufactures absorbent articles using the method for manufacturing absorbent articles of the present invention, and specifically the configuration of the manufacturing device up to the end of folding absorbent articles formed from integrated sheets;
FIG. 11 is a schematic flowchart of processes up to the point where absorbent articles formed from integrated sheets are folded and packaged;
FIG. 12 depicts a state in which a folding device has folded absorbent articles formed from integrated sheets in a direction orthogonal to a longitudinal direction of the absorbent articles (a width of the absorbent articles has been reduced in a direction orthogonal to the longitudinal direction of the absorbent articles);
FIG. 13 depicts a state in which a rotating device has rotated absorbent articles conveyed along the longitudinal direction of the absorbent articles by 90° such that the longitudinal direction of the absorbent articles aligns with a direction orthogonal to the conveyance direction of the absorbent articles;
FIG. 14 depicts a state in which, after the absorbent articles have been rotated by the rotating device, the absorbent articles in even-numbered rows counting upward from the lowest row (first row) in FIG. 13 have been moved from a first conveying path to a second conveying path;
FIG. 15 depicts a state in which position adjustment devices have reduced distances in the conveyance direction of absorbent articles conveyed on the first conveying path and the second conveying path;
FIG. 16 depicts a state in which absorbent articles, having been conveyed on the first conveying path and the second conveying path and supplied into folding devices, are folded in the longitudinal direction by the folding devices (the width of the absorbent articles is reduced in the longitudinal direction of the absorbent articles);
FIG. 17 depicts a state in which completely folded absorbent articles are collected onto a third conveying path and conveyed on the third conveying path;
FIG. 18 depicts a state in which absorbent articles conveyed on the third conveying path are placed between packaging sheets and are conveyed while placed between the packaging sheets;
FIG. 19 depicts a state in which edges of the packaging sheets have been sealed so that absorbent articles conveyed on the third conveying path are contained within sections independent from other absorbent articles;
FIG. 20 depicts a state in which packages, each containing an absorbent article, have been cut apart from packages containing other absorbent articles;
FIG. 21 depicts a state in which, when there are five rows of absorbent articles conveyed on the first conveying path, a rotating device has rotated absorbent articles conveyed along the longitudinal direction of the absorbent articles by 90° so that the longitudinal direction of the absorbent articles is aligned with a direction orthogonal to the conveyance direction of the absorbent articles;

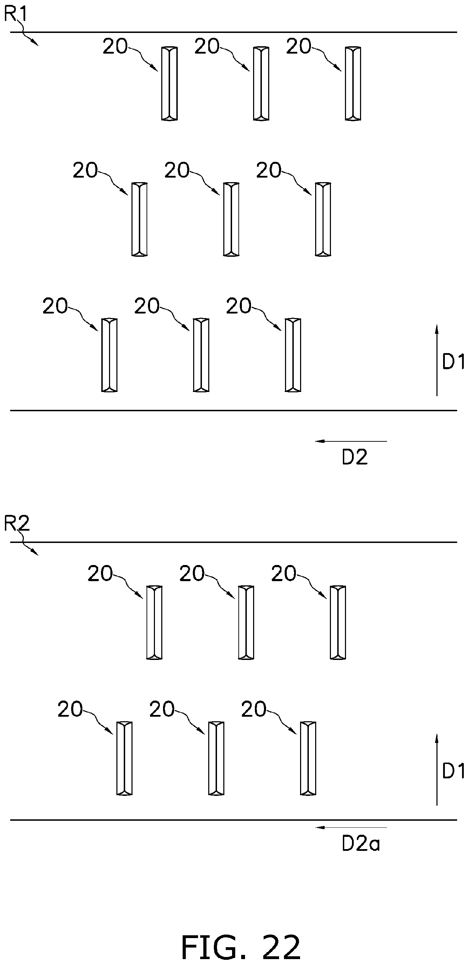

FIG. 22 depicts a state in which, after the absorbent articles have been rotated by the rotating device, the absorbent articles in even-numbered rows counting upward from the lowest row (first row) in FIG. 21 have been moved from a first conveying path to a second conveying path;
FIG. 23 relates to a method for manufacturing absorbent articles according to modification B and depicts a state in which eight rows of absorbent articles are conveyed on the first conveying path;
FIG. 24 depicts a state in which, in a case where the rows of absorbent articles are counted upward from the lowest row (first row) in FIG. 23, absorbent articles in the third and sixth rows have been moved from the first conveying path to the second conveying path, and absorbent articles in the second, fifth, and eighth rows have been moved from the first conveying path to a third conveying path;
FIG. 25 relates to a method for manufacturing absorbent articles of modification A and is a schematic flowchart of processes up to the point where absorbent articles formed from integrated sheets are folded and packaged;
FIG. 26 relates to modification A and shows a state in which multiple rows of absorbent articles formed from multiple rows of integrated sheets are conveyed on the first conveying path;
FIG. 27 depicts a state in which the absorbent articles in even-numbered rows, counting upward from the lowest row (first row) in FIG. 26, have been moved to the second conveying path, and the absorbent articles conveyed on the first conveying path and the second conveying path have been folded in a direction orthogonal to the longitudinal direction by folding devices;
FIG. 28 depicts a state in which a rotation and position adjustment device has rotated absorbent articles conveyed on the first conveying path and the second conveying path along the longitudinal direction of the absorbent articles by 90° so that the longitudinal direction is aligned with a direction orthogonal to the conveyance direction of the absorbent articles, and has reduced the distance between the absorbent articles in the conveyance direction; and
FIG. 29 is a schematic drawing of a configuration of a manufacturing device that manufactures absorbent articles using the manufacturing method of modification A, and specifically a configuration of the manufacturing device up to the point where absorbent articles formed from integrated sheets has been folded.

### DESCRIPTION OF EMBODIMENTS

Below is a description, made with reference to the drawings, of an embodiment of a method for manufacturing absorbent articles according to the present invention.

The embodiment described below is merely one embodiment of the present invention and does not limit the scope of the present invention. It should be understood that a person skilled in the art could make various changes to the following embodiment without departing from the spirit and scope of the present invention as set forth in the claims.

### (1) Absorbent article

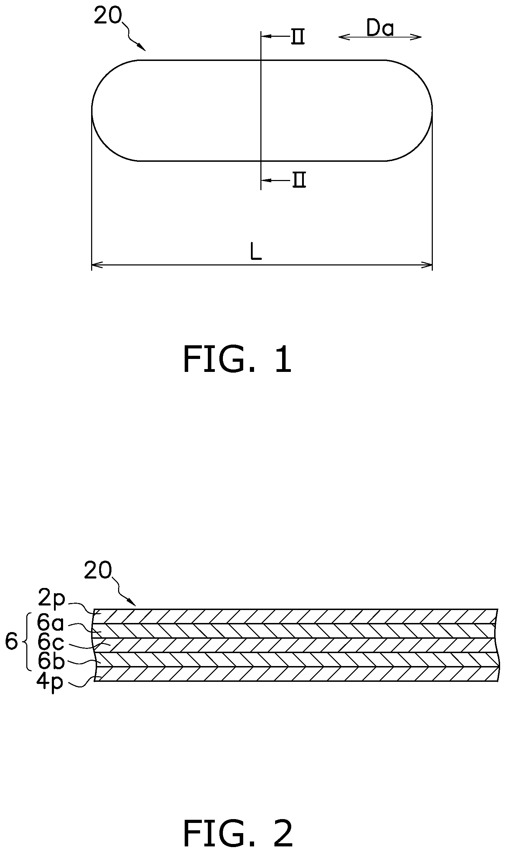

An absorbent article 20 manufactured using the method for manufacturing absorbent articles according to the present invention shall be described with reference to FIGS. 1 and 2. FIG. 1 is a diagram depicting an absorbent article 20 manufactured using the method for manufacturing absorbent articles of the present invention. Specifically, FIG. 1 depicts the absorbent article 20 before being folded in a longitudinal direction Da and a direction orthogonal to the longitudinal direction Da. FIG. 2 is a partial schematic cross-sectional view taken along arrow II-II of FIG. 1.

The absorbent article 20 absorbs and retains liquid. The absorbent article 20 is, for example, a sanitary napkin, a urine leakage pad, a disposable diaper, or the like. The absorbent article 20, such as a sanitary napkin, a urine leakage pad, or a disposable diaper, absorbs and retains bodily fluids discharged from a wearer using an absorber 6.

The absorbent article 20 is a sheet-form article having an oval shape, more specifically a rectangular shape with rounded corners, as shown in FIG. 1. The absorbent article 20 has a longitudinal direction Da. A length of the longitudinal direction Da of the absorbent article 20 is represented by the letter "L." The shape of the absorbent article 20 is not limited to the shape as depicted in FIG. 1. The shape of the absorbent article 20 may be, for example, a rectangular sheet-form article. In addition, the shape of the absorbent article 20 may be designed as appropriate depending on the use of the absorbent article 20, the function required of the absorbent article 20, etc

The absorbent article 20 mainly includes a sheet piece (top sheet) 2p cut out from a first sheet 2, a sheet piece (back sheet) 4p cut out from a second sheet 4, and the liquid-retaining absorber 6. The absorber 6 is arranged between the sheet piece 2p and the sheet piece 4p. The absorber 6, the sheet piece 2p, and the sheet piece 4p are joined at a predetermined position by, for example, an adhesive, and are integrated. Alternatively, the absorber 6, the sheet piece 2p, and the sheet piece 4p may be joined at a predetermined position by, for example, heat sealing or ultrasonic sealing, and may be integrated.

The absorbent article 20 does not need to include only of the sheet piece 2p of the first sheet 2, the sheet piece 4p of the second sheet 4, and the absorber 6. For example, the absorbent article 20 may include sheet pieces other than the sheet piece 2p of the first sheet 2, the sheet piece 4p of the second sheet 4, and the absorber 6. In addition, the absorbent article 20 may include tape or the like attached to the sheet piece 2p or the sheet piece 4p.

The sheet piece 2p is a surface that comes into contact with the skin of a wearer when the wearer wears the absorbent article 20. The first sheet 2 is a liquid-permeable sheet. The first sheet 2 is, for example, a nonwoven fabric. There are no limitations as to the material of the nonwoven fabric used as the first sheet 2; for example, synthetic fibers, recycled fibers, and natural fibers such as cotton can be used.

The second sheet 4 is a liquid-impermeable sheet. Liquid-impermeable in this case means that liquid permeability is relatively low, and is not limited to cases where liquid does not permeate at all. There are no limitations as to the material of the second sheet 4; for example, a laminated nonwoven fabric obtained by laminating a nonwoven fabric on a polyolefin resin sheet, a polyethylene sheet, or the like can be used.

The absorber 6 mainly includes an absorber core 6c, a sheet piece of a cover sheet 6a, and a sheet piece of a carrier sheet 6b. The absorber core 6c is arranged between the sheet piece of the cover sheet 6a and the sheet piece of the carrier sheet 6b. The cover sheet 6a and/or the carrier sheet 6b may be omitted from the absorber 6.

The absorber core 6c is formed of, for example, fluff pulp or air-laid nonwoven fabric. In addition, the absorber core 6c may include a water-absorbing, high-molecular-weight polymer.

The cover sheet 6a is arranged between the first sheet 2 and the absorber core 6c. The cover sheet 6a (a fourth sheet 60b, which is a raw material sheet for the cover sheet 6a) is formed of, for example, a hydrophilized nonwoven fabric. The cover sheet 6a diffuses liquid that has flowed in from the first sheet 2 side and spreads the liquid over a surface of the absorber core 6c, to make it easier for the liquid to be quickly absorbed into the absorber core 6c.

The carrier sheet 6b is arranged between the absorber core 6c and the second sheet 4. The carrier sheet 6b (a third sheet 60a, which is a raw material sheet for the carrier sheet 6b) is formed of, for example, a nonwoven fabric.

### (2) Method for manufacturing absorbent article

A specific example of a method for manufacturing the absorbent article 20 shall be described. The method for manufacturing the absorbent article 20 in this case includes steps of forming the absorbent article 20 from the first sheet 2, the second sheet 4, and the absorber 6, further processing the absorbent article 20, and finally manufacturing a package P containing the absorbent article 20.

### (2-1) Method for forming absorbent article

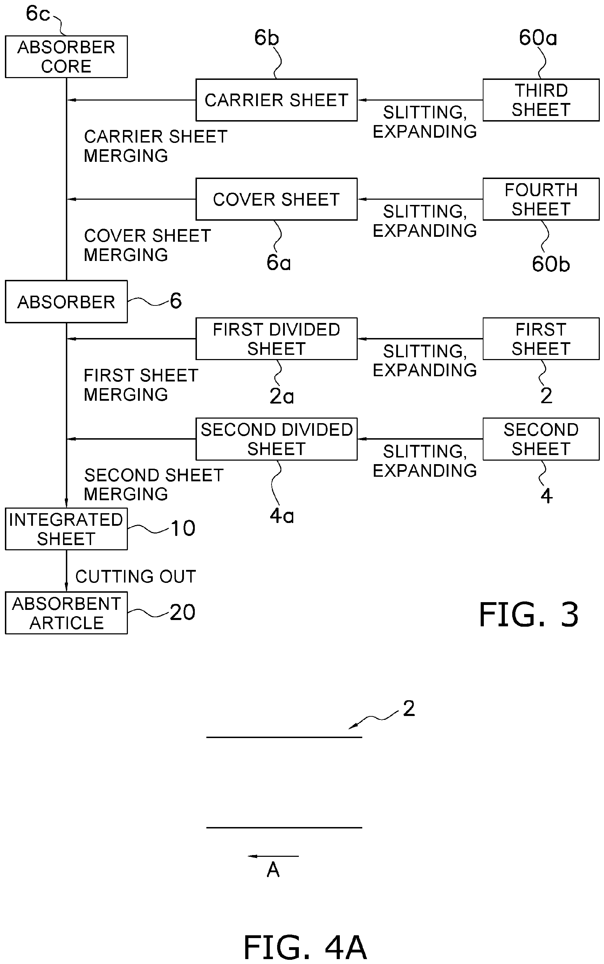

First, a method for forming the absorbent article 20 from the first sheet 2, the second sheet 4, and the absorber 6 shall be described on the basis of FIGS. 3 to 9.

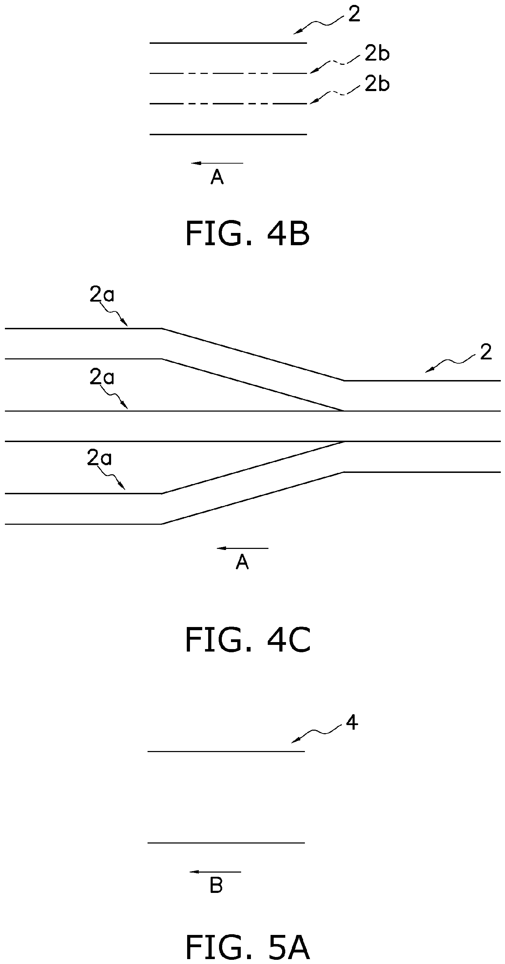

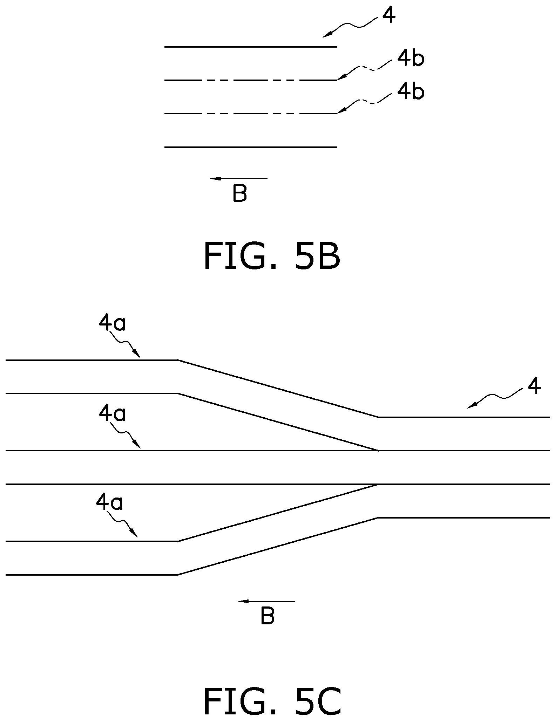

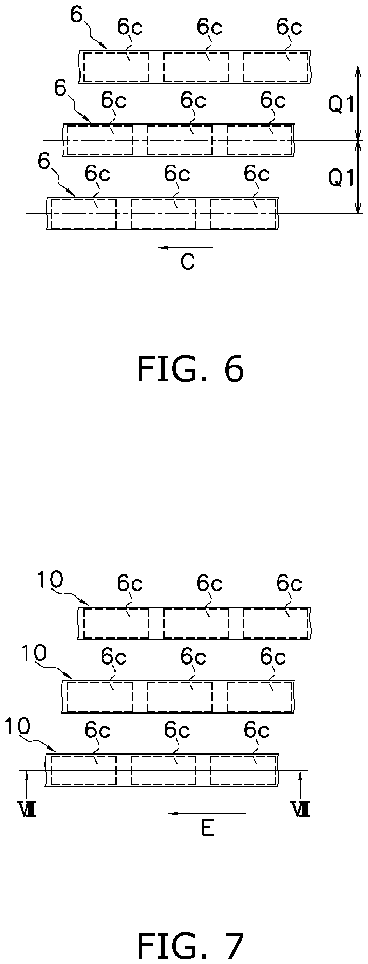

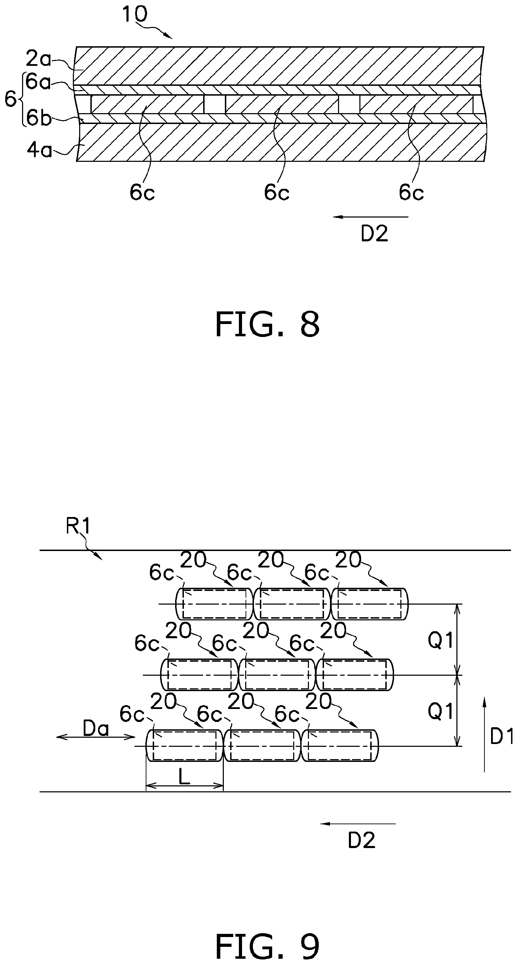

FIG. 3 is a diagram of a flow of processes for forming a before-folding absorbent article 20 from the first sheet 2, the second sheet 4, and the absorber 6. FIG. 4A depicts a first sheet 2 being conveyed. FIG. 4B depicts a state in which slits 2b have been formed in the conveyed first sheet 2 along a conveyance direction A of the first sheet 2. In FIG. 4B, the slits 2b are depicted as double-dashed lines. FIG. 4C depicts a state in which a conveying width of a plurality of first divided sheets 2a, having been divided by the slits 2b, is expanded in a direction orthogonal to the conveyance direction A of the first divided sheets 2a so as to increase a distance between adjacent first divided sheets 2a. FIG. 5A depicts a second sheet 4 being conveyed. FIG. 5B depicts a state in which slits 4b have been formed in the conveyed second sheet 4 along a conveyance direction B of the second sheet 4. In FIG. 5B, the slits 4b are depicted as double-dashed lines. FIG. 5C depicts a state in which a conveying width of a plurality of second divided sheets 4a, having been divided by the slits 4b, is expanded in a direction orthogonal to the conveyance direction B of the second divided sheets 4a so as to increase a distance between adjacent second divided sheets 4a. FIG. 6 depicts a state of conveying absorbers 6. FIG. 7 depicts a conveying state of sheets (referred to as integrated sheets 10) in which first divided sheets 2a, second divided sheets 4a, and absorbers 6 are integrated. FIG. 8 is a cross-sectional view taken along arrow VIII-VIII of FIG. 7. FIG. 9 shows a state in which multiple rows of absorbent articles 20 cut out from the multiple rows of integrated sheets 10 in FIG. 7 are conveyed on a first conveying path R1. In FIG. 9, to avoid complicating the drawing, only three absorbent articles 20 are depicted in the conveying direction of the absorbent articles 20. However, normally the absorbent articles 20 are conveyed on the first conveying path R1 in a state in which absorbent articles 20 are present on an upstream side and a downstream side of each absorbent article 20 in the conveying direction.

In forming an absorbent article 20, the absorber 6 is firstly formed, and processing described hereinafter is performed on the first sheet 2 and the second sheet 4.

The absorber 6 is formed as described below, mainly from the absorber core 6c, the cover sheet 6a, and the carrier sheet 6b.

Absorber cores 6c of absorbers 6, which is aligned in multiple rows in a direction orthogonal to the conveyance direction of the absorber cores, are conveyed in a predetermined conveyance direction by a conveying device (not shown). A third sheet 60a unfurled from web rolls (not shown) is divided in a direction orthogonal to a conveyance direction of the third sheet 60a to create multiple rows of carrier sheets 6b (divided third sheet 60a), and the absorber cores 6c of each row are arranged on the carrier sheets 6b of the corresponding row at predetermined intervals in the conveyance direction of the absorber cores 6c. The absorber cores 6c and the carrier sheets 6b are joined using, for example, an adhesive, heat sealing, or ultrasonic sealing. Furthermore, a fourth sheet 60b unfurled from web rolls (not shown) is divided in a direction orthogonal to a conveyance direction of the fourth sheet 60b to create multiple rows of cover sheets 6a (divided fourth sheet 60b), and the multiple absorber cores 6c of each row are covered by the cover sheets 6a of the corresponding row. The absorber cores 6c and the cover sheets 6a are joined using, for example, an adhesive, heat sealing, or ultrasonic sealing.

In this embodiment, the absorber cores 6c and the carrier sheets 6b are joined and the absorber cores 6c and the cover sheets 6a are joined, but the joint is not limited to such configurations. For example, instead of the carrier sheets 6b and the cover sheets 6a being joined to the absorber cores 6c, or in addition to the carrier sheets 6b and the cover sheets 6a being joined to the absorber cores 6c, the carrier sheets 6b and the cover sheets 6a may be joined with the absorber cores 6c sandwiched between the carrier sheets 6b and the cover sheets 6a. The integration achieved by joining the absorber cores 6c, the carrier sheets 6b, and the cover sheets 6a may be performed when the integrated sheets 10 are formed as described later. The sheet-form absorbers 6 formed in this manner are conveyed in the conveyance direction C (see FIG. 6) by a conveying device (not shown) in a state the sheet-form absorbers 6 being aligned in multiple rows in a direction orthogonal to a conveyance direction C. The conveying device (not shown) conveys the multiple rows of absorbers 6 with the positions of the absorber cores 6c conveyed in each row slightly shifted in the conveyance direction C.

The number of rows of absorbers 6 conveyed by the conveying device (not shown) is three in the present embodiment. However, the number of rows of absorbers 6 conveyed by the conveying device (not shown) is not limited to three, and may be two, four, or more.

The following processing is performed on the first sheet 2. The first sheet 2 is unfurled from a web roll (not shown) around which the first sheet 2 had been wound, and the first sheet 2 is conveyed in the conveyance direction A (see FIG. 4A). The slits 2b are then formed in the first sheet 2 along the conveyance direction A of the first sheet 2 by a cutting device (not shown), and the first sheet 2 is divided into multiple pieces (a plurality of first divided sheets 2a) in a direction orthogonal to the conveyance direction A (see FIG. 4B). For example, the first sheet 2 is divided into multiple pieces of the same width in a direction orthogonal to the conveyance direction A of the first sheet 2. In the example in FIG. 4B, the first sheet 2 is divided in three, but this example is not given by way of limitation; the number of divisions should be determined so as to be the same as the number of rows of absorbers 6. A conveying width of the plurality of first divided sheets 2a divided by the slits 2b is expanded in a direction orthogonal to the conveyance direction A of the first divided sheets 2a so as to increase a distance between adjacent first divided sheets 2a. An amount by which the conveying width of the plurality of first divided sheets 2a is expanded is determined such that a conveyed position of each row of the plurality of first divided sheets 2a overlaps a conveyed position of the absorbers 6 corresponding to those first divided sheets 2a.

The following processing is performed on the second sheet 4. The second sheet 4 is unfurled from a web roll (not shown) around which the second sheet 4 had been wound, and the second sheet 4 is conveyed in the conveyance direction B (see FIG. 5A). The slits 4b are then formed in the second sheet 4 along the conveyance direction B of the second sheet 4 by a cutting device (not shown), and the second sheet 4 is divided into multiple pieces (a plurality of second divided sheets 4a) in a direction orthogonal to the conveyance direction B (see FIG. 5B). For example, the second sheet 4 is divided into multiple pieces of the same width in a direction orthogonal to the conveyance direction B of the second sheet 4. In the example of FIG. 5B, the second sheet 4 is divided in three, but this example is not given by way of limitation; the number of divisions should be determined so as to be the same as the number of rows of absorbers 6. A conveying width of the plurality of second divided sheets 4a divided by the slits 4b is expanded in a direction orthogonal to the conveyance direction B of the second divided sheets 4a so as to increase a distance between adjacent second divided sheets 4a. An amount by which the conveying width of the plurality of second divided sheets 4a is expanded is determined such that a conveyed position of each row of the plurality of second divided sheets 4a overlaps the conveyed position of the absorbers 6 corresponding to those second divided sheets 4a.

After the absorbers 6, the first divided sheets 2a, and the second divided sheets 4a have been prepared in this manner, the integrated sheets 10 are formed as follows.

Of the multiple rows of first divided sheets 2a conveyed in the same direction as the conveyance direction C, the first divided sheet 2a of a corresponding row merges with each of the multiple rows of absorbers 6 conveyed in the conveyance direction C (see FIG. 3). Specifically, of the multiple rows of first divided sheets 2a conveyed in the same direction as the conveyance direction C, the first divided sheet 2a of a corresponding row covers each of the multiple rows of absorbers 6 conveyed in the conveyance direction C. Furthermore, of the multiple rows of second divided sheets 4a conveyed in the same direction as the conveyance direction C, the second divided sheet 4a of a corresponding row merges with each of the multiple rows of absorbers 6 covered by the first divided sheets 2a and conveyed in the conveyance direction C (see FIG. 3). Specifically, each of the multiple rows of absorbers 6 covered by the first divided sheets 2a and conveyed in the conveyance direction C is arranged on top of the second divided sheet 4a of the corresponding row of the multiple rows of second divided sheets 4a conveyed in the same direction as the conveyance direction C.

In other words, forming the integrated sheets 10 includes a step of arranging the absorber cores 6c, which are one example of absorbers, and a plurality of sheet members in a predetermined positional relationship. The plurality of sheet members include the cover sheets 6a (divided fourth sheet 60b), the carrier sheets 6b (divided third sheet 60a), the first divided sheets 2a, and the second divided sheets 4a. In other words, forming the integrated sheets 10 of the present embodiment includes a step of arranging the absorber cores 6c, which are one example of absorbers, and the divided sheets (first divided sheets 2a, second divided sheets 4a, carrier sheets 6b, and cover sheets 6a) formed by dividing raw material sheets (first sheet 2, second sheet 4, third sheet 60a, and fourth sheet 60b) in directions orthogonal to the conveyance directions of the raw material sheets. In the integrated sheets 10, the second sheet 4 (second divided sheets 4a), the carrier sheets 6b, the absorber cores 6c, the cover sheets 6a, and the first sheet 2 (first divided sheets 2a) are layered in the stated order as shown in FIG. 8.

The absorbers 6, the first divided sheets 2a, and the second divided sheets 4a are then joined using, for example, an adhesive, heat sealing, or ultrasonic sealing, resulting in the integrated sheets 10 (see FIGS. 3 and 7).

Multiple rows of integrated sheets 10, aligned in a direction orthogonal to a conveyance direction E, are conveyed in the conveyance direction E (see FIG. 7). The integrated sheet 10 of each row is cut by a cutting device (not shown) (for example, a die-cutting roll device) into a predetermined shape (a rounded rectangular shape in the present embodiment) such that the absorber cores 6c are arranged substantially in the center, and absorbent articles 20 are cut out so as to be aligned in multiple rows in a direction orthogonal to the conveyance direction E. In other words, the cutting device (not shown) forms absorbent articles 20 one after another from integrated sheets 10 aligned in multiple rows in a direction orthogonal to the conveyance direction E, the absorbent articles 20 being aligned in multiple rows in a direction orthogonal to the conveyance direction E and each having one absorber core 6c. In yet other words, the cutting device (not shown) cuts the first sheet 2 constituting the integrated sheets 10 to cut out sheet pieces 2p constituting the absorbent articles 20, and cuts the second sheet 4 constituting the integrated sheets 10 to cut out sheet pieces 4p constituting the absorbent articles 20. In addition, the cutting device (not shown) cuts out sheet pieces from the carrier sheets 6b constituting the integrated sheets 10, and cuts out sheet pieces from the cover sheets 6a.

In the manner described above, absorbent articles 20 are formed from the first sheet 2, the second sheet 4, and the absorbers 6.

### (2-2) Steps after the formation of absorbent articles

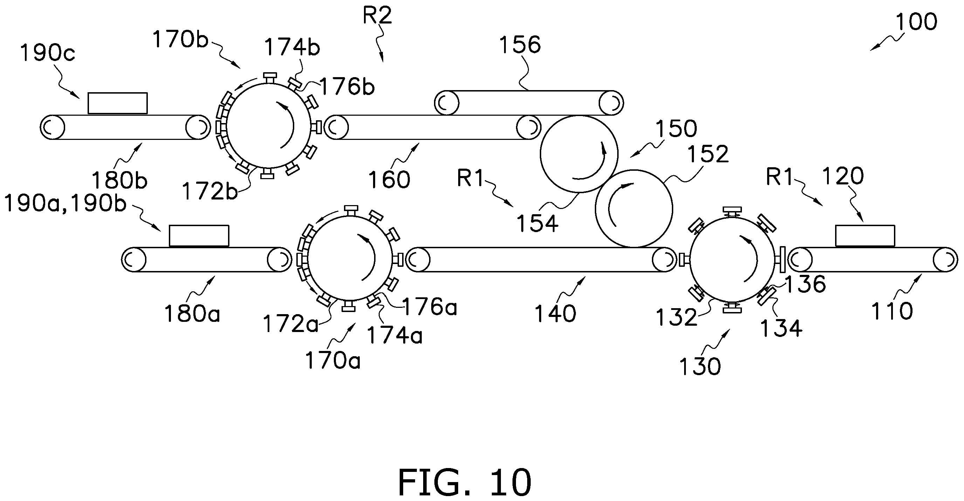

Next, the steps after the formation of absorbent articles 20 in the method for manufacturing absorbent articles 20 are explained with reference to FIGS. 10 to 20.

FIG. 10 is a schematic drawing of a configuration of a manufacturing device 100 that manufactures absorbent articles 20, and specifically a configuration of the manufacturing device 100 up to the point where absorbent articles 20 formed from integrated sheets are folded. The configuration of the manufacturing device 100 up to the end of folding absorbent articles 20 formed from integrated sheets mainly includes, as shown in FIG. 10, a conveying device 110, a folding device 120, a rotating device 130, a moving device 150, a first upstream conveying device 140, a second upstream conveying device 160, position adjustment devices 170a, 170b, a first downstream conveying device 180a, a second downstream conveying device 180b, and folding devices 190a, 190b, 190c.

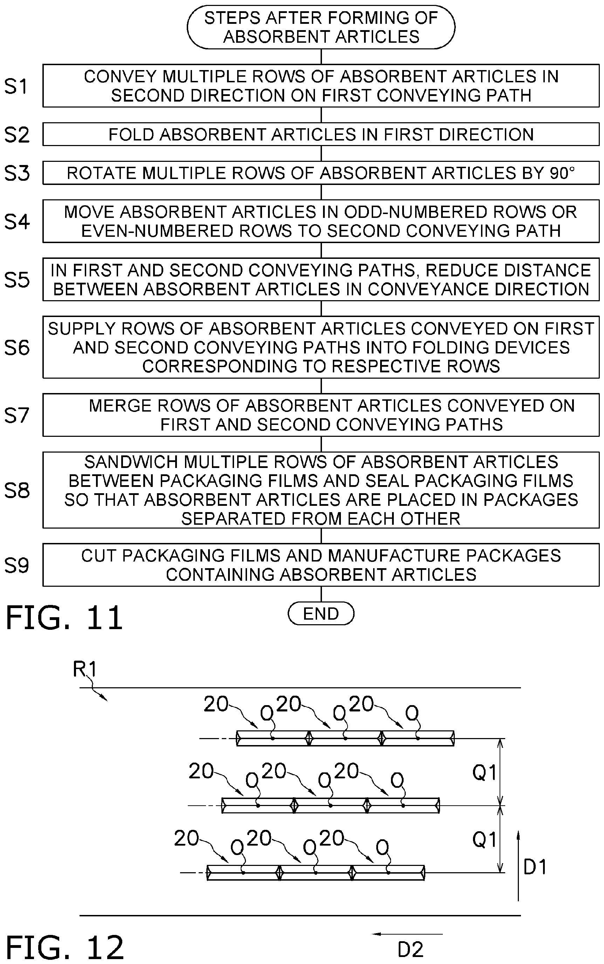

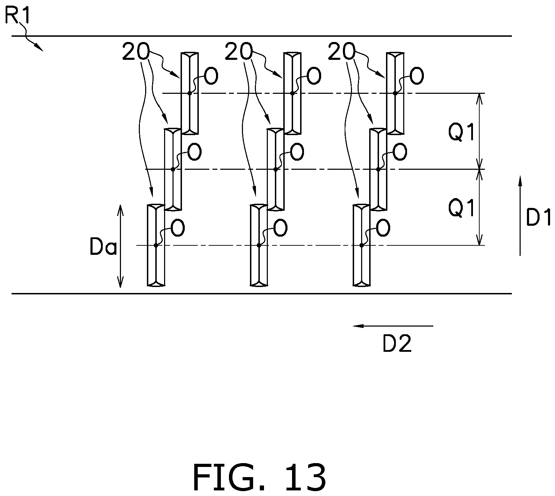

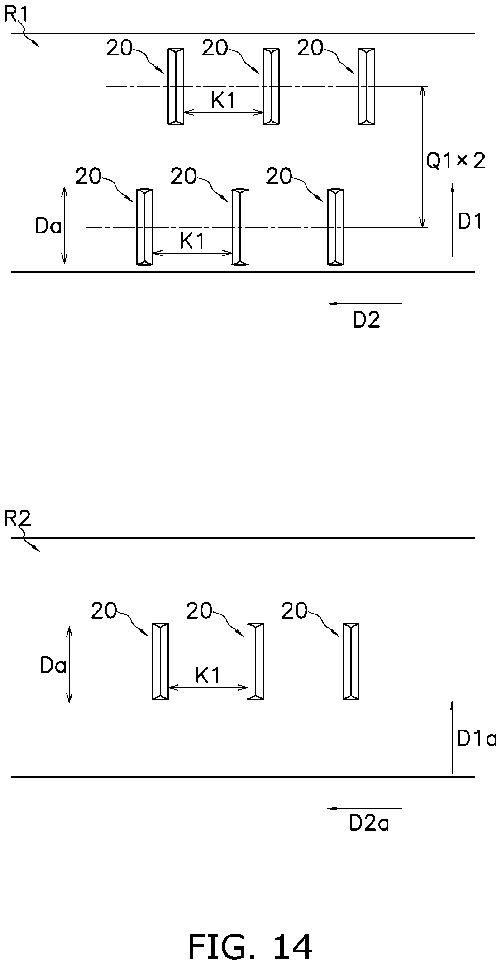

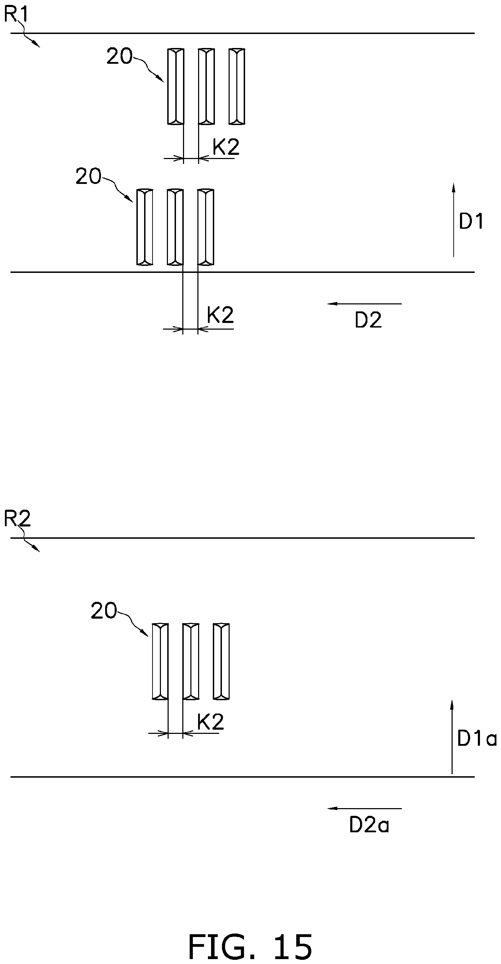

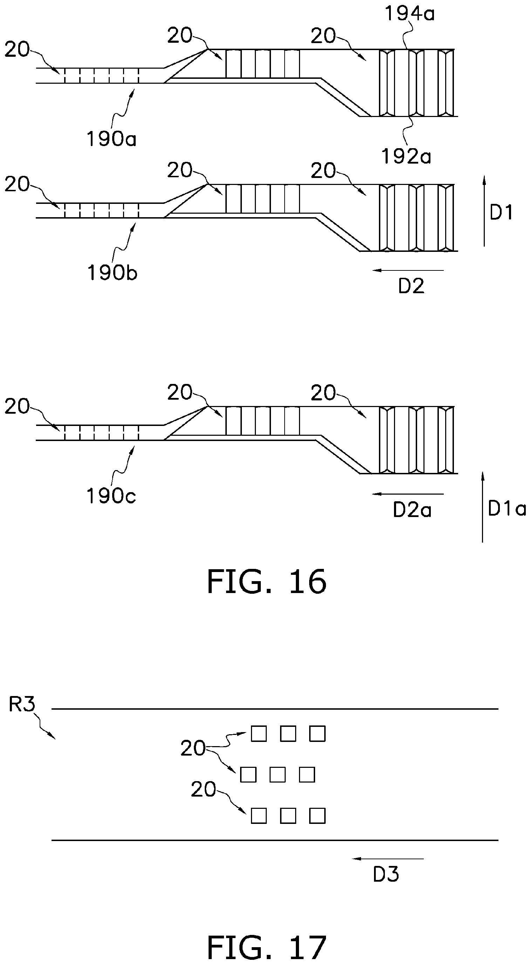

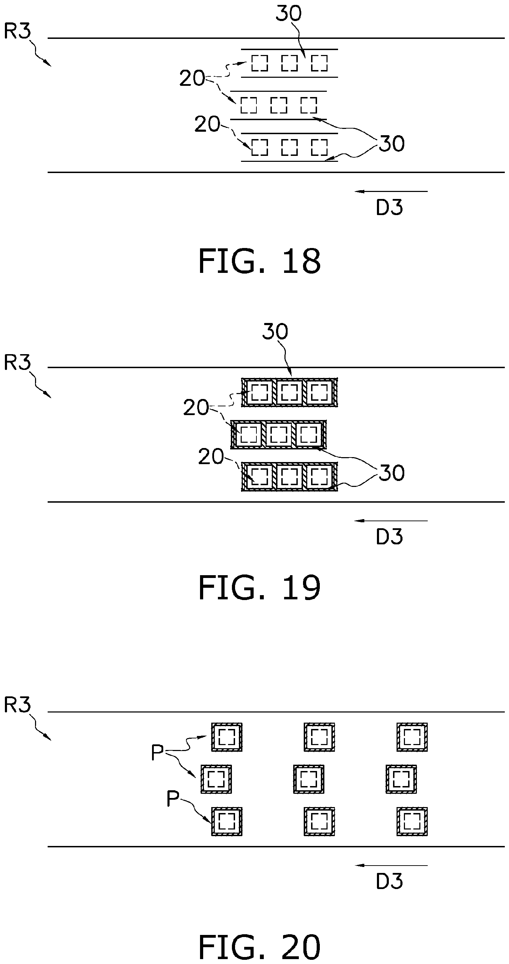

FIG. 11 is a schematic flowchart of processes up to the point where absorbent articles 20 formed from integrated sheets 10 are folded and packaged. FIG. 12 depicts a state in which the folding device 120 has folded absorbent articles 20 formed from integrated sheets 10 in a direction orthogonal to the longitudinal direction Da of the absorbent articles 20 (the width of the absorbent articles 20 has been reduced in a direction orthogonal to the longitudinal direction Da of the absorbent articles 20). FIG. 13 depicts a state in which the rotating device 130 has rotated absorbent articles 20 conveyed along the longitudinal direction Da of the absorbent articles 20 by 90° such that the longitudinal direction Da of the absorbent articles 20 aligns with a direction orthogonal to a second direction D2, which is the conveyance direction of the absorbent articles 20. FIG. 14 depicts a state in which, after the absorbent articles 20 have been rotated by the rotating device 130, the absorbent articles 20 in even-numbered rows, counting upward from the lowest row (first row) in FIG. 13, have been moved from the first conveying path R1 to a second conveying path R2 while remaining in the same state of alignment. FIG. 15 depicts a state in which the position adjustment device 170a has reduced a distance in the second direction D2, which is the conveyance direction of absorbent articles 20 conveyed on the first conveying path R1, and the position adjustment device 170b has reduced a distance in a second direction D2a, which is the conveyance direction of absorbent articles 20 conveyed on the second conveying path R2. FIG. 16 depicts a state in which absorbent articles 20, having been conveyed on the first conveying path R1 and the second conveying path R2 and supplied into the folding devices 190a, 190b, are folded in the longitudinal direction by the folding devices 190a, 190b (the width of the absorbent articles is reduced in the longitudinal direction of the absorbent articles). FIG. 17 depicts a state in which completely folded absorbent articles 20 are collected from the first conveying path R1 and the second conveying path R2 onto a third conveying path R3 and conveyed on the third conveying path R3. FIG. 18 depicts a state in which absorbent articles 20 conveyed on the third conveying path R3 are placed between packaging sheets 30 and are conveyed while placed between the packaging sheets 30. FIG. 19 depicts a state in which edges of the packaging sheets 30 have been sealed so that absorbent articles 20 conveyed along the third conveying path R3 are contained within sections independent from other absorbent articles 20. FIG. 20 depicts a state in which packages P, each containing an absorbent article 20, have been cut apart from packages P containing other absorbent articles 20. To avoid complicating the drawings in FIGS. 11 to 20, only a limited number of absorbent articles 20 are depicted in the conveyance direction of the absorbent articles 20. However, normally the absorbent articles 20 are conveyed in a state in which absorbent articles 20 are present on the upstream side and the downstream side of each absorbent article 20 in the conveying direction.

Multiple rows of absorbent articles 20 aligned in a direction orthogonal to the conveyance direction are conveyed on the first conveying path R1 by the conveying device 110. The conveying device 110 is, for example, a conveyor. The conveying device 110 conveys the absorbent articles 20 while holding the absorbent articles 20 on a conveying surface by suction using negative pressure so that the absorbent articles 20 do not lift off of the conveying surface. Multiple rows of absorbent articles 20 aligned in a first direction D1 are conveyed by the conveying device 110 on the first conveying path R1 in the second direction D2, which is orthogonal to the first direction D1 (see step S1 in FIG. 11, and FIG. 9). The conveying device 110 conveys the absorbent articles 20 in a state in which the longitudinal direction Da of the absorbent articles 20 is aligned with the second direction D2, which is the conveyance direction of the absorbent articles 20.

At this point in time, a distance Q1 (see FIG. 9) in the first direction D1 between rows of absorbent articles 20 conveyed on the first conveying path R1 is less than a length L of each absorbent article 20 in the longitudinal direction Da. The phrase "distance Q1 between rows in the first direction D1" means a distance between imaginary straight lines extending in the second direction D2 and passing through centers of the absorbent articles 20 of each row in the first direction D1.

The rows of absorbent articles 20 conveyed on the first conveying path R1 by the conveying device 110 are supplied into the folding device 120 corresponding to each row of absorbent articles 20. Absorbent articles 20 conveyed in the second direction D2 are folded by the folding device 120 in the first direction D1 (a direction orthogonal to the longitudinal direction Da of the absorbent articles 20) orthogonal to the second direction D2, which is the conveyance direction of the absorbent articles 20 (see step S2 and FIG. 12). A structure of the folding device 120 is the same as the folding devices 190a, 190b, 190c (described hereinafter), and will therefore be described within a description of a structure of the folding devices 190a, 190b, 190c.

The absorbent articles 20, having been folded by the folding device 120 in a direction orthogonal to the longitudinal direction Da of the absorbent articles 20, are rotated 90° by the rotating device 130. In other words, the absorbent articles 20 conveyed in a state in which the longitudinal direction Da of the absorbent articles 20 is aligned with the second direction D2, which is the conveyance direction of the absorbent articles 20, are rotated by the rotating device 130 so that the longitudinal direction Da of the absorbent articles 20 will be orthogonal to the conveyance direction of the absorbent articles 20 (will be aligned with the first direction D1) (see step S3 and FIG. 13).

The rotating device 130 has a structure such as the following, which is not provided by way of limitation.

The rotating device 130 has a drum 132, a plurality of arms 136, and suction pads 134 provided at end parts of the arms 136. The drum 132 is cylindrical, and the drum 132 rotates about a center axis of the cylindrical drum 132. The plurality of arms 136 extend radially outward from the cylindrical drum 132. The arms 136 are cylindrical, and the arms 136 rotate about center axes of the cylindrical arms 136. The plurality of arms 136 are placed on an outer surface of the drum 132 at equal intervals in a circumferential direction. The suction pads 134, which suction-hold the absorbent articles 20, are provided at the end parts (radially outer ends) of the arms 136. The suction pads 134 suction-hold the absorbent articles 20 by negative pressure. Suction surfaces, by which the suction pads 134 suction-hold the absorbent articles 20, have approximately the same shape and size as the absorbent articles 20. In other words, the suction pads 134 also have a longitudinal direction.

The rotating device 130 is placed adjacent to the conveying device 110, downstream of the conveying device 110 in the conveyance direction. In addition, the rotating device 130 is placed adjacent to the first upstream conveying device 140, upstream of the first upstream conveying device 140 in the conveyance direction. When the arms 136 of the rotating device 130 pass near the conveying device 110, the suction pads 134 provided at the end parts of the arms 136 receive absorbent articles 20 conveyed by the conveying device 110. The arms 136 pass near the first upstream conveying device 140 due to the rotation of the drum 132. At this time, the absorbent articles 20 held by the suction pads 134 of the arms 136 are delivered to the first upstream conveying device 140.

The arms 136 rotate about the center axes of the cylindrical arms 136 in accordance with the rotation of the drum 132. Specifically, when the arms 136 pass near the conveying device 110, the longitudinal direction of the suction pads 134 provided at the end parts of the arms 136 approximately aligns with the circumferential direction of the cylindrical drum 132. As a result of the arms 136 rotating about the center axes of the cylindrical arms 136 in accordance with the rotation of the drum 132, when the arms 136 pass near the first upstream conveying device 140, a direction orthogonal to the longitudinal direction of the suction pads 134 provided at the end parts of the arms 136 is approximately aligned with the circumferential direction of the cylindrical drum 132. As a result, absorbent articles 20 conveyed by the conveying device 110 in a state in which the longitudinal direction Da of the absorbent articles 20 is aligned with the second direction D2, which is the conveyance direction of the absorbent articles 20, are rotated so that the longitudinal direction Da of the absorbent articles 20 will be orthogonal to the conveyance direction of the absorbent articles 20 (will be aligned with the first direction D1), and are delivered to the first upstream conveying device 140.

The rotating device 130 rotates each absorbent article 20 about a center point O thereof. The center points O of the absorbent articles 20 are points in the absorbent articles 20 conveyed by the conveying device 110, where imaginary center lines of the absorbent articles 20 in the first direction D1 and imaginary center lines of the absorbent articles 20 in the second direction D2 intersect. The absorbent articles 20 conveyed in each row are positionally offset in the second direction D2 as shown in FIG. 11 to avoid the absorbent articles 20, which have been rotated about the center points O, overlapping rotated absorbent articles 20 conveyed in adjacent row.

The first upstream conveying device 140 receives the absorbent articles 20 rotated by the rotating device 130 and conveys the absorbent articles 20 along the first conveying path R1. The first upstream conveying device 140 is, for example, a conveyor. The first upstream conveying device 140 conveys the absorbent articles 20 while holding the absorbent articles 20 on a conveying surface by suction using negative pressure so that the absorbent articles 20 do not lift off of the conveying surface. Multiple rows of absorbent articles 20 aligned along the first direction D1 are conveyed on the first conveying path R1 by the first upstream conveying device 140 in the second direction D2, which is orthogonal to the first direction D1 (step S1 of FIG. 11). The first upstream conveying device 140 conveys the absorbent articles 20 in a state in which the longitudinal direction Da of the absorbent articles 20 is orthogonal to the conveyance direction of the absorbent articles 20.

The absorbent articles 20 conveyed by the first upstream conveying device 140 are supplied into a folding device (denoted by the symbol " 190") downstream as shall be described hereinafter. However, since the rows of absorbent articles 20 are in a state of overlapping adjacent rows of absorbent articles 20 in the first direction D1 as in FIG. 13, there is a risk that when the absorbent articles 20 conveyed in the state of FIG. 13 are to be supplied into the corresponding folding device, the supplying of the absorbent articles 20 into the folding device will be hindered by the absorbent articles 20 conveyed in adjacent rows.

Therefore, some of the rows of absorbent articles 20 conveyed on the first conveying path R1 by the first upstream conveying device 140 are moved to the second conveying path R2, which is different from the first conveying path R1, using the moving device 150. Counting along the first direction D1 from the row of absorbent articles 20 farthest upstream in the first direction D1 (the lowest row in FIG. 13) as a reference, odd-numbered rows or even-numbered rows of absorbent articles 20 are moved by the moving device 150 as rows of absorbent articles 20 of divided groups to the second conveying path R2, which is different from the first conveying path R1, while remaining in the same state of alignment in the first direction D1 (see FIG. 14, and step S4 of FIG. 11). For example, in the present embodiment, counting along the first direction D1 using the row of absorbent articles 20 farthest upstream in the first direction D1 as a reference, even-numbered rows of absorbent articles 20 are moved by the moving device 150 as rows of absorbent articles 20 of divided groups to the second conveying path R2, which is different from the first conveying path R1, while remaining in the same state of alignment in the first direction D1.

As a result of this configuration, the width between adjacent rows of the absorbent articles 20 conveyed on the conveying paths can be increased without expanding the widths of the conveying paths conveying the rows of the absorbent articles 20. For example, in the example depicted in FIGS. 13 and 14, before the rows of the absorbent articles 20 of divided groups are moved to the second conveying path R2 by the moving device 150, the distance between rows of the absorbent articles 20 in the first conveying path R1 is Q1, whereas after the rows of the absorbent articles 20 of divided groups have been moved to the second conveying path R2 by the moving device 150, the distance between rows of the absorbent articles 20 in the first conveying path R1 is Q1×2. In particular, if the distance Q1×2 between rows of the absorbent articles 20 in the first conveying path R1 after the rows of the absorbent articles 20 of divided groups have been moved by the moving device 150 is longer than the length L of the absorbent articles 20 in the longitudinal direction Da, it is possible to reduce the possibility that the absorbent articles 20 will be kept from being supplied into the folding devices by the absorbent articles 20 conveyed on adjacent rows.

The phrase "while remaining in the same state of alignment in the first direction D1" means that when a row of absorbent articles 20 is moved from the first conveying path R1 to the second conveying path R2, the alignment of the absorbent articles 20 is not changed, and the number of rows in the first direction D1 (in other words, a direction orthogonal to the conveyance direction of the absorbent articles 20) is not increased or reduced.

For example, in the examples shown in FIGS. 13 and 14, counting along the first direction D1 from the row of absorbent articles 20 farthest upstream in the first direction D1 as a reference, the even-numbered row of absorbent articles 20 (one row) is moved by the moving device 150 from the first conveying path R1 to the second conveying path R2 without increasing the number of rows (such that absorbent articles 20 are aligned in one row along the conveyance direction).

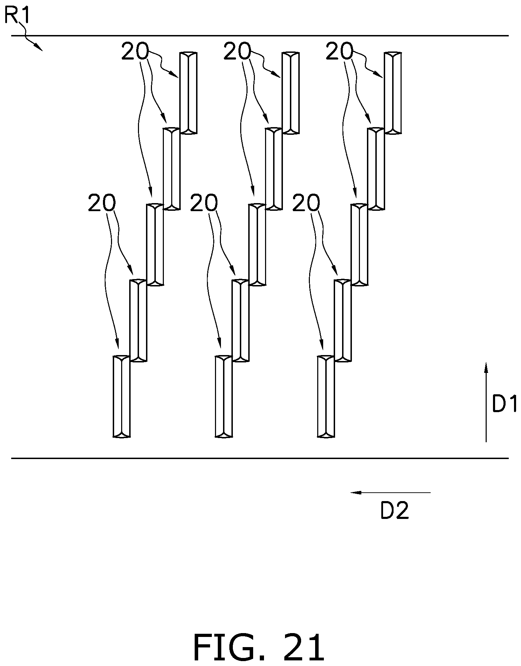

For example, as shown in FIG. 21, when the first upstream conveying device 140 originally conveys five rows of absorbent articles 20 and even-numbered rows (the second and fourth rows) of absorbent articles 20, counting along the first direction D1 from the row of absorbent articles 20 farthest upstream in the first direction D1 as a reference, are moved to the second conveying path R2 by the moving device 150, the moving device 150 moves the absorbent articles 20 from the first conveying path R1 to the second conveying path R2 without increasing or reducing the number of rows (the number of rows remains two).

In addition, when odd-numbered rows or even-numbered rows of absorbent articles 20, counting along the first direction D1 from the row of absorbent articles 20 farthest upstream in the first direction D1 as a reference, are moved by the moving device 150 as rows of absorbent articles 20 of divided groups from the first conveying path R1 to the second conveying path R2, the moving device 150 preferably does not change the distance between the rows of absorbent articles 20 in the first direction D1.

Some of the rows of absorbent articles 20 conveyed on the first conveying path R1 by the first upstream conveying device 140 are moved to the second conveying path R2, which is different from the first conveying path R1, by, for example, a plurality of cylindrical rotating drums 152 and 154, and a conveyor 156, such as are shown in FIG. 10. The rotating drums 152 and 154 are substantially equal in width to the first upstream conveying device 140 in a direction orthogonal to the conveyance direction of the absorbent articles 20. The rotating drum 152 is placed such that when the rotating drum 152 is rotating, an outer surface thereof is substantially in contact with a conveying surface of the first upstream conveying device 140. The rotating drum 154 is placed such that when the rotating drum 154 is rotating, an outer surface thereof is substantially in contact with the outer surface of the rotating drum 152. In addition, the rotating drum 154 is placed such that when the rotating drum 154 is rotating, the outer surface thereof is substantially in contact with a conveying surface of a conveyor 156.

When the manufacturing device 100 is in operation, the rotating drums 152 and 154 rotate in directions shown by arrows in FIG. 10. Rollers of the conveyor 156 also rotate in directions shown by arrows in FIG. 10.

One or more suction holes for suctioning the absorbent articles 20 by negative pressure are provided at positions in the outer surface of the rotating drum 152 substantially in contact with the conveying surface of the first upstream conveying device 140 where the suction holes will come into contact with the absorbent articles 20 conveyed from the first conveying path R1 to the second conveying path R2. As a result of this configuration, absorbent articles 20 conveyed on the conveying surface of the first upstream conveying device 140 move to the outer surface of the rotating drum 152 upon coming into contact with the outer surface of the rotating drum 152. In order for the absorbent articles 20 to be delivered smoothly, the suctioning of the moved absorbent articles 20 by the conveying surface of the first upstream conveying device 140 is preferably stopped when the absorbent articles 20 are moved by the moving device 150.

Though not described in detail, after this, in the same way, absorbent articles 20 that had been suctioned to the outer surface of the rotating drum 152 move to the outer surface of the rotating drum 154 upon coming into contact with the outer surface of the rotating drum 154, and absorbent articles 20 that had been suctioned to the outer surface of the rotating drum 154 move to the conveying surface of the conveyor 156 upon coming into contact with the conveying surface of the conveyor 156. Eventually, counting along the first direction D1 from the farthest upstream row of absorbent articles 20 in the first direction D1 as a reference, odd-numbered rows or even-numbered rows of absorbent articles 20 (even-numbered rows in the present embodiment) are moved by the moving device 150 as rows of absorbent articles 20 of divided groups to the second upstream conveying device 160.

The second upstream conveying device 160 receives the absorbent articles 20 from the moving device 150 and conveys the absorbent articles 20 along the second conveying path R2. The second upstream conveying device 160 conveys the absorbent articles 20 while suctioning the absorbent articles 20 by negative pressure so that the absorbent articles 20 do not lift off of the conveying surface. The absorbent articles 20 aligned in multiple rows along the first direction D1a are conveyed by the second upstream conveying device 160 on the second conveying path R2, in the second direction D2a orthogonal to the first direction D1a (see FIG. 14). The second upstream conveying device 160 conveys the absorbent articles 20 in a state in which the longitudinal direction Da of the absorbent articles 20 is orthogonal to the conveyance direction of the absorbent articles 20.

Preferably, the first upstream conveying device 140 and the second upstream conveying device 160 are arranged at positions of different heights. In other words, the first conveying path R1 in which the first upstream conveying device 140 conveys the absorbent articles 20 and the second conveying path R2 in which the second upstream conveying device 160 conveys the absorbent articles 20 are preferably arranged at least partially at positions of different heights. By arranging the first conveying path R1 and the second conveying path R2 at least partially at positions of different heights, space can be effectively utilized in the height direction and the area in which the manufacturing device 100 is installed is readily reduced. It is more preferable for the first conveying path R1 and the second conveying path R2 to be arranged in at least partially overlapping positions in a plan view.

Next, a step is performed in which, in each row of absorbent articles 20 conveyed in the first conveying path R1 and the second conveying path R2, the distance between absorbent articles 20 in the conveyance direction of the absorbent articles 20 is reduced from a distance K1 to a distance K2 (see step S5 and FIG. 15). By reducing the distance between absorbent articles 20 in the conveyance direction of the absorbent articles 20, it is possible to simultaneously perform downstream processes (e.g., steps for manufacturing packages P containing the absorbent articles 20, described hereinafter) on a plurality of absorbent articles 20. Therefore, the manufacturing steps can be made more efficient and the manufacturing device 100 can be simplified.

The distance between the absorbent articles 20 in the conveyance direction of the absorbent articles 20 is adjusted by the position adjustment devices 170a, 170b. The position adjustment device 170a corresponds to the first conveying path R1, and the position adjustment device 170b corresponds to the second conveying path R2. The position adjustment device 170a and the position adjustment device 170b have different alphabet letters "a" and "b" added to the end of the symbols to make description more convenient, but these devices have the same structure and function. Therefore, the position adjustment device 170a shall be described here and a description of the position adjustment device 170b shall be omitted.

The position adjustment device 170a has, for example, a structure such as the following.

The position adjustment device 170a has a drum 172a, a plurality of arms 176a, and suction pads 174a provided at end parts of the arms 176a. The drum 172 is cylindrical, and the drum 172 rotates about a center axis of the cylindrical drum 172. The plurality of arms 176 extend radially outward from the cylindrical drum 172. A plurality of the arms 176 are placed around a circumferential direction of the drum 172. The arms 176 are able to move relative to the drum 172 along the circumferential direction of the drum 172, and a distance between adjacent arms 176 can be adjusted as depicted in FIG. 10. The suction pads 174, which suction-hold the absorbent articles 20, are provided at the end parts (radially outer ends) of the arms 176. The suction pads 174 suction-hold the absorbent articles 20 by negative pressure. Suction surfaces, by which the suction pads 174 suction-hold the absorbent articles 20, have approximately the same shape and size as the absorbent articles 20.

The position adjustment device 170a is placed adj acent to the first upstream conveying device 140, downstream of the first upstream conveying device 140 in the conveyance direction. In addition, the position adjustment device 170a is placed adjacent to the first downstream conveying device 180a, upstream of the first downstream conveying device 180a in the conveyance direction. When the arms 176 of the rotating device 170 pass near the first upstream conveying device 140, the suction pads 174 provided at the end parts of the arms 176 receive absorbent articles 20 conveyed by the first upstream conveying device 140. The arms 176 pass near the first downstream conveying device 180a due to the rotation of the drum 172. At this time, the absorbent articles 20 held by the suction pads 174 of the arms 176 are delivered to the first downstream conveying device 180a.

The arms 176a move in the circumferential direction of the drum 172 in accordance with the rotation of the drum 172, such that the distance between adjacent arms 176a reaches a predetermined distance in the circumferential direction of the drum 172. As a result, the distance K2 between the absorbent articles 20 conveyed by the first downstream conveying device 180a is smaller than the distance K1 between the absorbent articles 20 conveyed by the first upstream conveying device 140.

The first downstream conveying device 180a and the second downstream conveying device 180b are conveyors that convey the absorbent articles 20 while applying suction using negative pressure, so that the absorbent articles 20 do not lift off of the conveying surfaces. The first downstream conveying device 180a receives the absorbent articles 20 from the position adjustment device 170a and conveys rows of the absorbent articles 20 in the first conveying path R1. The second downstream conveying device 180b receives the absorbent articles 20 from the position adjustment device 170b and conveys rows of the absorbent articles 20 in the second conveying path R2. The first downstream conveying device 180a and the second downstream conveying device 180b convey rows of the absorbent articles 20 in a direction orthogonal to the longitudinal direction Da of the absorbent articles 20, and supply pluralities of the absorbent articles 20 into the folding devices 190a, 190b, 190c corresponding to the respective rows of absorbent articles 20 among the folding devices 190a, 190b, 190c (see FIG. 16, step S6 of FIG. 11). Specifically, among the rows of the absorbent articles 20 conveyed to the first downstream conveying device 180a, the absorbent articles 20 in the row depicted at the top of FIG. 15 are supplied into the folding device 190a. Among the rows of absorbent articles 20 conveyed to the first downstream conveying device 180a, the absorbent articles 20 in the row depicted at the bottom of FIG. 15 are supplied into the folding device 190b. The row of absorbent articles 20 conveyed to the second downstream conveying device 180b is supplied to the folding device 190c.

The folding device 190a has a structure such as follows. The structures and functions of the folding devices 190b, 190c are the same as the structure and function of the folding device 190a, and are therefore not described to avoid duplicate descriptions. The previously mentioned folding device 120 also has a structure and function similar to that of the folding device 190a.

The folding device 190a has a side wall 192a placed on the left side and a side wall 192b placed on the right side, when the device is viewed along the conveyance direction of the absorbent articles 20 supplied into the folding device 190a. The side wall 192a has a tapered part 194a for reducing a conveyance width of the absorbent articles 20.

The tapered part 194a is formed as a curved surface so as to guide left end parts of the absorbent articles 20 supplied into the folding device 190a. The tapered part 194a raises the left end parts of the absorbent articles 20 supplied into the folding device 190a, and folds the absorbent articles 20 so that the left end parts of the absorbent articles 20 are placed near the centers of the absorbent articles 20 in a direction orthogonal to the conveyance direction of the absorbent articles 20.

A tapered part 194b is formed as a curved surface so as to guide right end parts of the absorbent articles 20 supplied into the folding device 190a. The tapered part 194b raises the right end parts of the absorbent articles 20 supplied into the folding device 190a, and folds the absorbent articles 20 so that the right end parts of the absorbent articles 20 are placed near the centers of the absorbent articles 20 in a direction orthogonal to the conveyance direction of the absorbent articles 20.

By folding the absorbent articles 20 using the tapered part 194a of the side wall 192a and the tapered part 194b of the side wall 192b, the width of the absorbent articles 20 in the longitudinal direction Da decreases, and the absorbent articles 20 will be sheet-form articles folded into substantially square shapes at the point in time when the absorbent articles 20 finish being folded in the folding device 190a.

Next, the manufacturing device 100 causes the absorbent articles 20 folded by the folding devices 190a, 190b and conveyed on the first conveying path R1, and the absorbent articles 20 folded by the folding device 190c and conveyed on the second conveying path R2, to merge (step S7 of FIG. 11). The manufacturing device 100 conveys rows of merged absorbent articles 20 on the third conveying path R3.

The third conveying path R3 may be the first conveying path R1 or the second conveying path R2, or may be another path independent of the first conveying path R1 and the second conveying path R2. For example, in one example, the rows of absorbent articles 20 conveyed on the second conveying path R2 are moved between two rows of absorbent articles 20 conveyed on the first conveying path R1, using a device (not shown) similar to the moving device 150. In other words, the rows of absorbent articles 20 conveyed on the second conveying path R2 are moved in a direction orthogonal to the conveyance direction of the absorbent articles 20 to the same positions as when the absorbent articles 20 had originally been conveyed on the first conveying path R1, using a device (not shown) similar to the moving device 150. The rows of absorbent articles 20 merged in the third conveying path R3 (the first conveying path R1) in this manner are conveyed on the third conveying path R3 in a third direction D3 (see FIG. 17).

Next, packaging sheets 30 are supplied to the rows of absorbent articles 20 conveyed on the third conveying path R3 so as to sandwich the absorbent articles 20 conveyed along the third conveying path R3 (see FIG. 18). Furthermore, the packaging sheets 30 are sealed so that the absorbent articles 20 are placed in spaces independent of each other. The method for sealing the packaging sheets 30 may include using an adhesive, or may include heat sealing or ultrasonic sealing (see step S8 of FIG. 11, and FIG. 19). In FIG. 19, hatching is added to the sealing locations.

Furthermore, the sealed packaging sheets 30 are cut at appropriate positions so that packages P, each containing absorbent articles 20, are manufactured (step S9 of FIG. 11).

The absorbent articles 20 are manufactured according to the steps described above. The method for manufacturing absorbent articles 20 described above is merely one example; changes can be made as appropriate as long as there are no inconsistencies.

For example, the steps for manufacturing the absorbent articles 20 may include steps other than those described above, e.g., steps such as attaching tape to outer surfaces of the absorbent articles 20.

In addition, for example, the steps for manufacturing the absorbent articles 20 need not include some of the steps described above. The folding step in step S2 may be omitted in cases such as when, for example, there is no need to reduce the width of the absorbent articles 20 in a direction orthogonal to the longitudinal direction Da.

In addition, for example, the steps for manufacturing the absorbent articles 20 described above include a step of merging the rows of absorbent articles 20 conveyed on the first conveying path R1 and the second conveying path R2, but this example is not provided by way of limitation. The merging step of step S7 may be omitted in cases such as when, for example, the rows of absorbent articles 20 conveyed on the first conveying path R1 and the second conveying path R2 are packaged by separated devices.

In addition, for example, in the manufacturing device 100 described above, the absorbent articles 20 moved by the moving device 150 are delivered to the second upstream conveying device 160 and then delivered to the position adjustment device 170b. However, this example is not provided by way of limitation; the absorbent articles 20 moved by the moving device 150 may be delivered directly to the position adjustment device 170b and then delivered to the second downstream conveying device 180b.

### (3) Characteristics

Characteristics of the method for manufacturing absorbent articles 20 of the present application shall be described below.

(3-1)
The method for manufacturing absorbent articles 20 of the present embodiment includes a conveying step (step S1 of FIG. 10), a moving step (step S4 of FIG. 10), and a supplying step (step S6 of FIG. 10). In the conveyance step, absorbent articles 20 aligned in multiple rows along a first direction D1 are conveyed on a first conveyance path R1 in a second direction D2 orthogonal to the first direction D1. In the moving step, odd-numbered rows or even-numbered rows of absorbent articles 20, when counted along the first direction D1 from the row of absorbent articles 20 farthest upstream in the first direction D1 as a reference, are moved to a second conveying path R2, which is different from the first conveying path R1, as rows of absorbent articles 20 of divided groups while remaining in the same state of alignment in the first direction D1. The supplying step is performed after the rows of the absorbent articles 20 of divided groups have been moved to the second conveying path R2. In the supplying step, the rows of absorbent articles 20 are conveyed in a direction orthogonal to a longitudinal direction of the absorbent articles 20 in the first conveying path R1 and the second conveying path R2, and are each supplied into a folding device 190a, 190b, or 190c corresponding to the respective row of absorbent articles 20 among a plurality of devices 190a, 190b, 190c for folding the absorbent articles 20. The folding devices 190a, 190b, 190c are one example of the processing devices in the claims.

In the above embodiment, the first direction D1 is represented by an upward arrow in FIG. 9, but this direction is not a limitation on the first direction D1. For example, in FIG. 9, the first direction D1 may be represented by a downward arrow.

In this method for manufacturing absorbent articles 20, absorbent articles aligned in multiple rows in the first direction D1 orthogonal to the second direction D2, which is a conveyance direction, are separated into odd-numbered rows and even-numbered rows, and are then supplied into devices for processing the absorbent articles 20, which correspond to respective rows of the absorbent articles 20. Therefore, even if a distance between the rows of absorbent articles 20 is not expanded, space around each row of absorbent articles 20 is readily ensured when the absorbent articles 20 are supplied into the processing devices. As a result, it is possible to suppress the incidence of events in which absorbent articles 20 in adjacent rows impede absorbent articles 20 from being supplied into the processing device of each row, without expanding the width at which the absorbent articles 20 are conveyed.

(3-2)
In the method for manufacturing absorbent articles 20 of the present embodiment, a distance Q1 in the first direction D1 between rows of absorbent articles 20 conveyed on the first conveying path R1, before the rows of absorbent articles 20 of divided groups are moved to the second conveying path R2, is less than a length L of each absorbent article 20 in the longitudinal direction Da.

Due to this configuration, the width of the first conveying path R1 (the width in a direction orthogonal to the conveyance direction) is suppressed, and the area in which the device 100 for manufacturing the absorbent articles 20 is installed can be suppressed.

(3-3)
The method for manufacturing absorbent articles 20 of the present embodiment includes a step (step S3 of FIG. 11) in which the absorbent articles 20, conveyed in a state in which the longitudinal direction Da of the absorbent articles 20 is aligned with the conveyance direction of the absorbent articles 20, are rotated so that the longitudinal direction Da of the absorbent articles 20 is orthogonal to the conveyance direction of the absorbent articles 20.

Due to this configuration, the width of the first conveying path R1 (the width in a direction orthogonal to the conveyance direction) is suppressed and the area in which the manufacturing device 100 is installed can be suppressed than in cases in which the absorbent articles 20 are constantly conveyed in a state in which the longitudinal direction Da of the absorbent articles 20 is orthogonal to the conveyance direction of the absorbent articles 20.

(3-4)
In the method for manufacturing absorbent articles 20 of the present embodiment, the step of rotating the absorbent articles 20 is a step in which the absorbent articles 20 conveyed on the first conveying path R1 in a state in which the longitudinal direction Da of the absorbent articles 20 and the second direction D2 are aligned are rotated, before the rows of absorbent articles 20 of divided groups are moved to the second conveying path R2, so that the longitudinal direction Da of the absorbent articles 20 aligns with the first direction D1.

Due to the step of rotating the absorbent articles 20 being performed before the rows of absorbent articles 20 of divided groups are moved to the second conveying path R2, there is no need to provide separate rotating devices 130 for the absorbent articles 20 conveyed on the first conveying path R1 and the absorbent articles 20 conveyed on the second conveying path R2. Therefore, increase in the installation area of the manufacturing device 100 or the cost of the manufacturing device 100 can be suppressed by using this manufacturing method.

(3-5)
The method for manufacturing absorbent articles 20 of the present embodiment includes, after the step of rotating the absorbent articles 20, a step of reducing a width between the absorbent articles 20 in the conveyance direction of the absorbent articles 20, in each row of the absorbent articles 20 (step S5 of FIG. 11).

By placing the absorbent articles 20 close together, it is possible to simultaneously perform downstream processes (e.g., steps for manufacturing packages P containing the absorbent articles 20, as in step S8 and step S9 of FIG. 11) on a plurality of absorbent articles 20. As a result, the manufacturing steps can be made more efficient, and the manufacturing device 100 can be simplified.

(3-6)
In the method for manufacturing absorbent articles 20 of the present embodiment, the first conveying path R1 and the second conveying path R2 are arranged at least partially at positions of different heights.

Due to this configuration, space can be effectively utilized in the height direction as well, and the area in which the manufacturing device 100 is installed is readily reduced.

(3-7)
The method for manufacturing absorbent articles 20 of the above embodiment is a method for manufacturing absorbent articles 20 that have an absorber 6, a sheet piece (including a sheet piece 2p, a sheet piece 4p, a sheet piece of a cover sheet 6a, and a sheet piece of a carrier sheet 6b) arranged adjacent to the absorber 6. The method for manufacturing absorbent articles 20 includes a forming step, an arrangement step, a joining step, and a cutting-out step. In the forming step, at least one first raw material sheet among a plurality of conveyed raw material sheets is divided into a plurality in a direction orthogonal to a conveyance direction of the raw material sheet, and a plurality of divided sheets are formed. In the present embodiment, all of the plurality of raw material sheets (first sheet 2, second sheet 4, third sheet 60a, and fourth sheet 60b) are divided into a plurality in a direction orthogonal to the conveyance direction of the raw material sheets, and a plurality of divided sheets are formed. In the arrangement step, a plurality of sheet members and absorber cores 6c, serving as an example of absorbers, are arranged. The plurality of sheet members include the first divided sheets 2a formed from the first sheet 2, the second divided sheets 4a formed from the second sheet 4, the carrier sheets 6b formed from the third sheet 60a, and the cover sheets 6a formed from the fourth sheet 60b. In the arrangement step, the absorber cores 6c are arranged between at least two sheet members. In the present embodiment, the second divided sheets 4a, the carrier sheets 6b, the absorber cores 6c, the cover sheets 6a, and the first divided sheets 2a are layered in the stated order. In the joining step, the absorber cores 6c and the sheet members corresponding to the absorber cores 6c are connected. The joining of the absorber cores 6c and the opposing sheet members includes not only a mode in which the absorber cores 6c and the sheet members are directly joined using an adhesive or heat sealing, but also included cases in which sheet members sandwiching the absorber cores 6c therebetween are joined using an adhesive or heat sealing so that the absorber cores 6c and the sheet members come to be contacted together. In addition, the joining of the absorber cores 6c and the opposing sheet members includes cases in which the absorber cores 6c and a sheet member opposing the absorber cores 6c come to be firmly adhered together via the other sheet member. In the cutting-out step, the sheet members joined to the absorber cores 6c are cut and the sheet pieces are cut out.

In this method for manufacturing absorbent articles 20, a step of arranging the absorber cores 6c and the sheet members including the divided sheets in a predetermined positional relationship is performed after the first sheet 2, the second sheet 4, the third sheet 60a, and the fourth sheet 60b, which are raw material sheets, have been divided into a plurality in a direction orthogonal to the conveyance direction of the raw material sheets and a plurality of divided sheets have been formed. Therefore, it is possible for the incidence of events where inappropriate force is applied locally to raw material sheets when the raw material sheet is cut and the sheet members constituting the absorbent articles become wrinkled or twisted, to be suppressed compared to a case where the raw material sheets is divided into a plurality in a direction orthogonal to the conveyance direction of the raw material sheets after the absorber cores 6c and the raw material sheets have been arranged adjacent to each other.

### (4) Modifications

Modifications of the above embodiment shall be described below. The following modifications may be combined, as appropriate, as long as the modifications are not inconsistent with each other.

### (4-1) Modification A

In the above embodiment, the step of rotating the absorbent articles 20 by 90° (step S3 of FIG. 11) is executed before the step of moving the rows of absorbent articles 20 of divided groups to the second conveying path R2 (step S4 of FIG. 11).

However, the order of steps described in the above embodiment is merely one example; the order of the step of moving the rows of absorbent articles 20 of divided groups to the second conveying path R2 and the step of rotating the absorbent articles 20 by 90° may be reversed. In this case, the positions of the absorbent articles 20 of the rows conveyed on the first conveying path R1 may be aligned in the second direction D2 (the absorbent articles 20 need not be offset in the second direction D2).

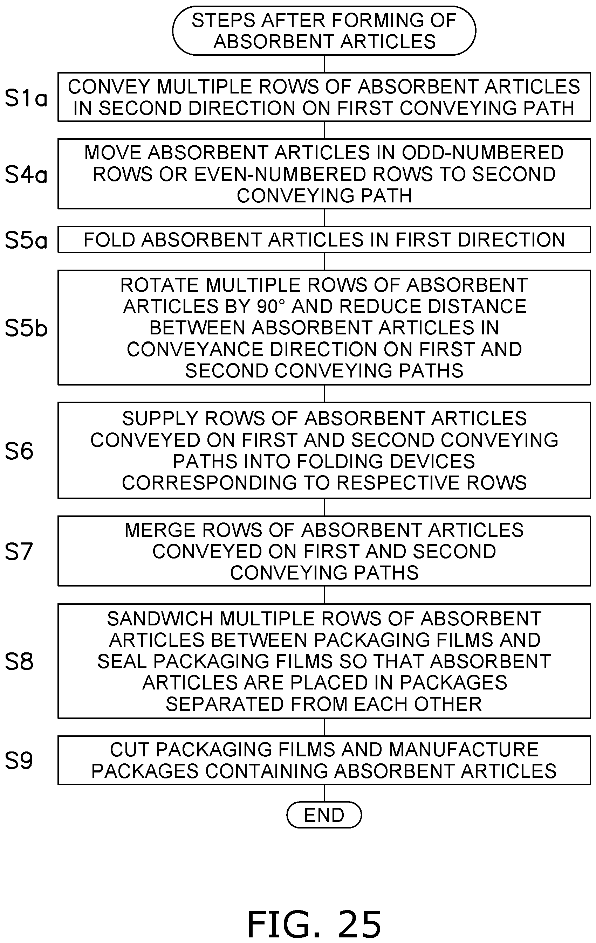

A specific example of a method for manufacturing absorbent articles 20 in modification A and a manufacturing device 300 used in this manufacturing method shall be described with reference to FIGS. 25 to 29. The method for manufacturing absorbent articles 20 in modification A and the manufacturing device 300 have many of the same features as the method for manufacturing absorbent articles 20 in the above embodiment and the manufacturing device 100. To avoid duplicate descriptions, the description shall primarily focus on the differences and the similar features shall essentially not be described.

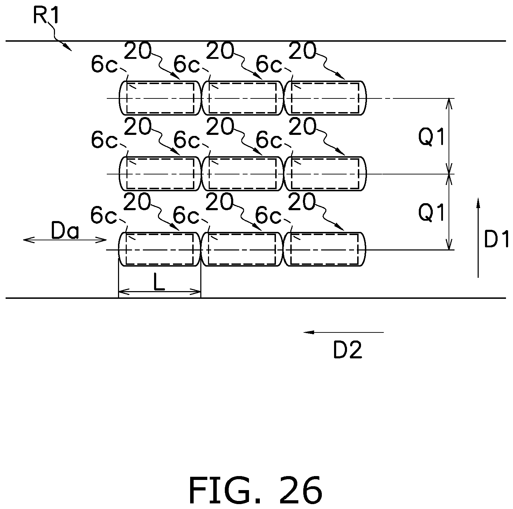

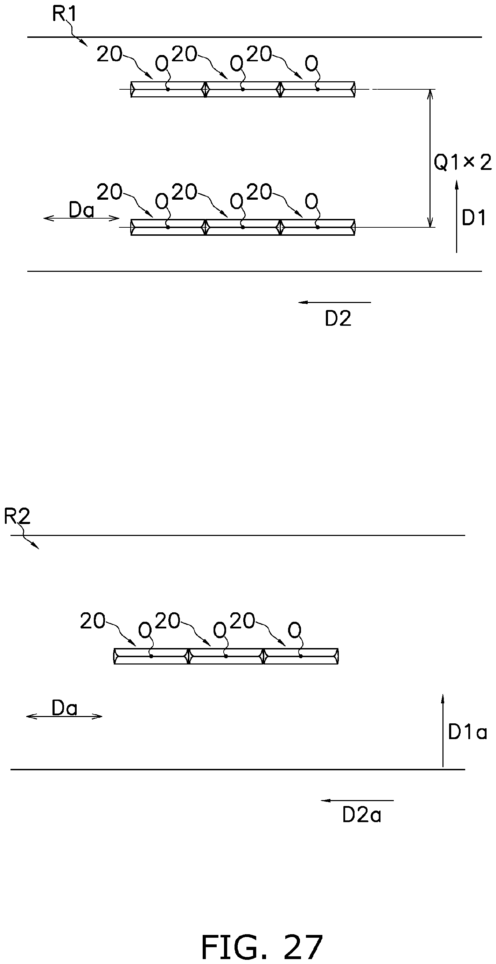

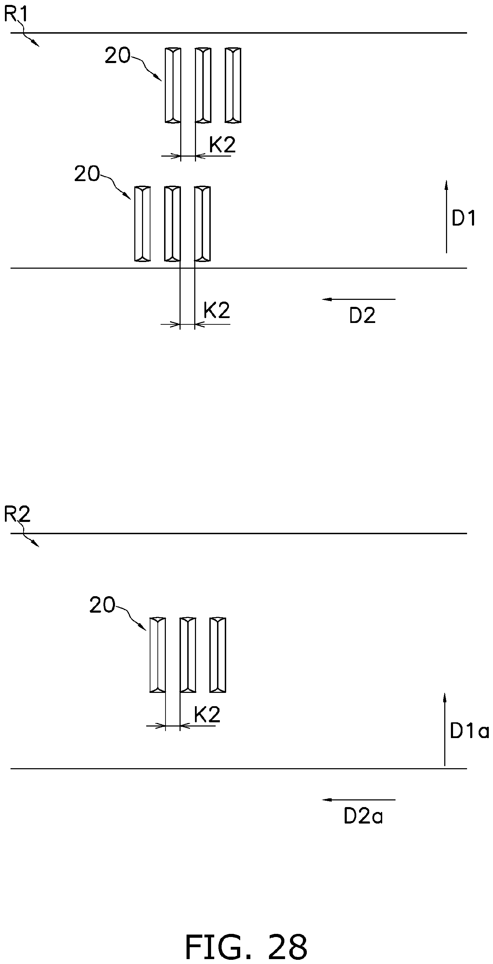

FIG. 25, pertaining to the method for manufacturing absorbent articles 20 of modification A, is a schematic flowchart of processes up to the point where absorbent articles 20 formed from integrated sheets 10 are folded and packaged. FIG. 26, pertaining to modification A, shows a state in which multiple rows of absorbent articles 20 formed from multiple rows of integrated sheets 10 are conveyed on the first conveying path R1. FIG. 27 depicts a state in which the absorbent articles 20 in even-numbered rows, counting upward from the lowest row (first row) in FIG. 26, have been moved from the first conveying path R1 to the second conveying path R2. FIG. 27 depicts a state after the absorbent articles 20 conveyed on the first conveying path R1 and the second conveying path R2 have been folded in a direction orthogonal to the longitudinal direction Da by folding devices 332a and 332b. FIG. 28 depicts a state in which a rotation and position adjustment device 340 has rotated absorbent articles 20 conveyed on the first conveying path R1 and the second conveying path R2 along the longitudinal direction Da of the absorbent articles 20, by 90°, so that the longitudinal direction Da is aligned with a direction orthogonal to the second direction D2, which is the conveyance direction of the absorbent articles 20, and has reduced the distance between the absorbent articles 20 in the second direction D2.

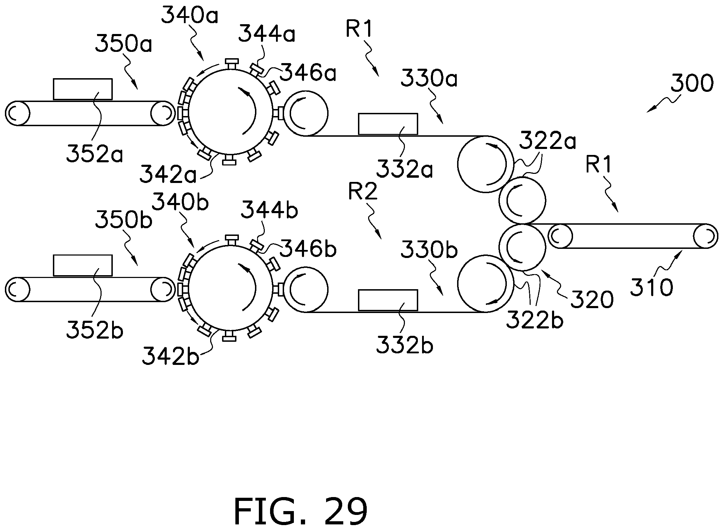

FIG. 29 is a schematic drawing of a configuration of a manufacturing device 300 that manufactures absorbent articles 20 using the manufacturing method of modification A. Specifically, FIG. 29 is a schematic drawing of a configuration of the manufacturing device 300 up to the point where absorbent articles 20 formed from integrated sheets 10 has been folded. The configuration of the manufacturing device 300 mainly includes, as shown in FIG. 29, a first conveying device 310, a branching device 320, second conveying devices 330a and 330b, folding devices 332a and 332b, rotation and position adjustment devices 340a and 340b, third conveying devices 350a and 350b, and folding devices 352a and 352b.

The method for manufacturing absorbent articles 20 of modification A shall now be described.

Absorbent articles 20 in multiple rows aligned in a direction orthogonal to the conveyance direction are conveyed on the first conveying path R1 by the first conveying device 310. The first conveying device 310 is, for example, a conveyor. Specifically, on the first conveying path R1, the absorbent articles 20 aligned in multiple rows along the first direction D1 are conveyed by the first conveying device 310 in the second direction D2, which is orthogonal to the first direction D1 (step S1a of FIG. 25). As shown in FIG. 26, the first conveying device 310 conveys the absorbent articles 20 in a state in which the longitudinal direction Da of the absorbent articles 20 is aligned with the second direction D2, which is the conveyance direction of the absorbent articles 20.

At this point in time, as in the above embodiment, a distance Q1 (see FIG. 26) in the first direction D1 between rows of absorbent articles 20 conveyed on the first conveying path R1 is preferably less than a length L of each absorbent article 20 in the longitudinal direction Da.

Next, in modification A, the manufacturing device 300, unlike in the above embodiment, causes the conveying path of the absorbent articles 20 to branch into two paths without folding the absorbent articles 20 in a direction orthogonal to the longitudinal direction Da or rotating the absorbent articles 20 (in a state in which the longitudinal direction Da and the second direction D2 are aligned). In other words, in modification A, the manufacturing device 300 moves some rows of absorbent articles 20 among the multiple rows of absorbent articles 20 conveyed on the first conveying path R1 to the second conveying path R2 in a state in which the longitudinal direction Da and the second direction D2 are aligned (step S4a of FIG. 25).

Specifically, the branching device 320 of the manufacturing device 300 moves the absorbent articles 20 of odd-numbered rows and even-numbered rows when counted along the first direction D1 from the farthest upstream row of absorbent articles 20 in the first direction D1 (the lowest row in FIG. 26) as a reference, respectively, to different conveying paths while remaining in the same state of alignment in the first direction D1. The path to which the branching device 320 moves the absorbent articles 20 of odd-numbered rows is defined as the first conveying path R1 along with the conveying path used by the first conveying device 310. The path to which the branching device 320 moves the absorbent articles 20 of even-numbered rows (sometimes referred to as the absorbent articles 20 of divided groups) is defined as the second conveying path R2, which is different from the first conveying path R1. This definition is for the sake of convenience; the path to which the branching device 320 moves the absorbent articles 20 of even-numbered rows may be defined as the first conveying path R1 along with the conveying path used by the first conveying device 310, and the path to which the branching device 320 moves the absorbent articles 20 of odd-numbered rows may be defined as the second conveying path R2, which is different from the first conveying path R1.

The branching device 320 has, for example, a plurality of rollers 322a and 322b in which suction holes (not shown) through which air is drawn into interiors of the rollers are formed in outer peripheral surfaces. The rollers 322a are used to move the absorbent articles 20 to the first conveying path R1, and the rollers 322b are used to move the absorbent articles 20 to the second conveying path R2. In the present modification A, suction holes (not shown) are formed in the outer peripheral surfaces of the rollers 322a at positions corresponding to the absorbent articles 20 of odd-numbered rows conveyed on the first conveying device 310 in the first direction D1. Suction holes (not shown) are formed in the outer peripheral surfaces of the rollers 322b at positions corresponding to the absorbent articles 20 of even-numbered rows conveyed on the first conveying device 310 in the first direction D1. For example, the rollers 322a and 322b placed as shown in FIG. 29 rotate as shown by the arrows in FIG. 29 while suctioning air through the suction holes, whereby the absorbent articles 20 of odd-numbered rows are moved to the second conveying device 330a, which constitutes part of the first conveying path R1, and the absorbent articles 20 of even-numbered rows are moved to the second conveying device 330b, which constitutes part of the first conveying path R1.

As a result of the absorbent articles 20 of odd-numbered rows being moved to the second conveying device 330a and the absorbent articles 20 of even-numbered rows being moved to the second conveying device 330b, the width between adj acent rows of the absorbent articles 20 conveyed on the conveying paths can be increased without increasing the widths of the conveying paths conveying the rows of the absorbent articles 20. For example, in the example depicted in FIGS. 26 and 27, before the rows of the absorbent articles 20 of divided groups are moved to the second conveying path R2 by the branching device 320, the distance between rows of the absorbent articles 20 in the first conveying path R1 is Q1, whereas after the rows of the absorbent articles 20 of divided groups have been moved to the second conveying path R2 by the branching device 320, the distance between rows of the absorbent articles 20 in the first conveying path R1 is Q1×2. In particular, when the distance Q1×2 between rows of the absorbent articles 20 in the first conveying path R1 after the rows of the absorbent articles 20 of divided groups have been moved to the second conveying path R2 by the branching device 320 is longer than the length L of the absorbent articles 20 in the longitudinal direction Da, it is possible to reduce the possibility that the absorbent articles 20 will be kept from being supplied into the folding devices by the absorbent articles 20 conveyed on adjacent rows.

The process of steps S5a, S5b, and S6 in FIG. 25 shall be described below using as an example the process performed on the absorbent articles 20 conveyed on the first conveying path R1. To avoid duplicate descriptions, the process performed on the absorbent articles 20 conveyed on the second conveying path R2 shall not be described. Following description can be applied to the process performed on the absorbent articles 20 conveyed on the second conveying path R2 by replacing the symbol "a" used to denote equipment with the symbol "b." The process of steps S7-S9 in FIG. 25 is the same as the process of steps S7-S9 of FIG. 11 in the above embodiment, and therefore shall not be described.

The absorbent articles 20 moved to the second conveying device 330a by the branching device 320 are conveyed on the first conveying path R1 by the second conveying device 330a. The second conveying device 330a is, for example, a conveyor. The second conveying device 330a conveys the absorbent articles 20 on a conveying surface while applying suction using negative pressure, so that the absorbent articles 20 do not lift off of the conveying surface. The second conveying device 330a conveys the absorbent articles 20 in a state in which the longitudinal direction Da of the absorbent articles 20 is aligned with the second direction D2, which is the conveyance direction of the absorbent articles 20.

The rows of absorbent articles 20 conveyed on the first conveying path R1 by the second conveying device 330a are supplied into the folding device 332a corresponding to those rows of absorbent articles 20. The absorbent articles 20 conveyed in the second direction D2 are folded by the folding device 332a in the first direction D1 (a direction orthogonal to the longitudinal direction Da of the absorbent articles 20) orthogonal to the second direction D2, which is the conveyance direction of the absorbent articles 20 (step S5a of FIG. 25). A structure of the folding device 332a is similar to the structure of the previously described folding device 120, and therefore shall not be described.

The absorbent articles 20 folded by the folding device 332a in a direction orthogonal to the longitudinal direction Da of the absorbent articles 20 are conveyed to the rotation and position adjustment device 340a. The rotation and position adjustment device 340a is a device in which the rotating device 130 and the position adjustment devices 170a, 170b of the above embodiment are combined and integrated.

The rotation and position adjustment device 340a rotates the absorbent articles 20 by 90°. Specifically, the rotation and position adjustment device 340a rotates the absorbent articles 20 conveyed in a state in which the longitudinal direction Da of the absorbent articles 20 is aligned with the second direction D2, which is the conveyance direction of the absorbent articles 20 so that the longitudinal direction Da of the absorbent articles 20 is orthogonal to the conveyance direction (second direction D2) of the absorbent articles 20 (so that the longitudinal direction Da is aligned with the first direction D1) (see step S5b of FIG. 26, and FIG. 28).

The rotation and position adjustment device 340a adjusts the distance between the absorbent articles 20 in the conveyance direction (second direction D2) of the absorbent articles 20 while rotating the absorbent articles 20 at the same time (see step S5b of FIG. 26, and FIG. 28). It is not only cases in which both processes are performed literally at the same time that the rotation and position adjustment device 340a rotates the absorbent articles 20 and at the same time adjusts the distance between the absorbent articles 20 in the conveyance direction (second direction D2) of the absorbent articles 20. The rotation and position adjustment device 340a rotating the absorbent articles 20 and at the same time adjusting the distance between the absorbent articles 20 in the conveyance direction (second direction D2) of the absorbent articles 20 means that rotation of the absorbent articles 20 and adjustment of the distance between the absorbent articles 20 are both performed by the rotation and position adjustment device 340a (even if the timings are different). Preferably, the rotation and position adjustment device 340a executes rotation of the absorbent articles 20 and adjustment of the distance between the absorbent articles 20 in the conveyance direction (second direction D2) of the absorbent articles 20 such that the timings of both processes at least partially overlap.

The adjustment of the distance between the absorbent articles 20 shall be described in detail.

If the rotation and position adjustment device 340a were to only rotate the absorbent articles 20, the absorbent articles 20 would be separated from each other by a distance K1 in the conveyance direction (second direction D2), as shown in FIG. 14. In contrast, the adjustment of the distance between the absorbent articles 20 in the conveyance direction (second direction D2) of the absorbent articles 20, which is performed by the rotation and position adjustment device 340a, is a process of shortening the distance K1 between the absorbent articles 20 to a distance K2 (< K1) (see FIG. 28). By shortening the distance between the absorbent articles 20 in the conveyance direction of the absorbent articles 20, the downstream processes (e.g., the steps of manufacturing the packages P containing the absorbent articles 20, described hereinafter) can be performed at the same time on a plurality of absorbent articles 20. Therefore, the manufacturing steps can be made more efficient, and the manufacturing device 100 can be simplified.

A simple description of the structure and operations of the rotation and position adjustment device 340a shall be given here because the rotation and position adjustment device 340a has a configuration similar to that of the rotating device 130 and the position adjustment devices 170a, 170b.

The rotation and position adjustment device 340a has a drum 342a, a plurality of arms 346a, and suction pads 344a provided at end parts of the arms 346a. The drum 342a is cylindrical, and the drum 342a rotates about a center axis of the cylindrical drum 342a. The plurality of arms 346a extend radially outward from the cylindrical drum 342a. A plurality of the arms 346a are placed around a circumferential direction of the drum 342a. The suction pads 344a, which suction-hold the absorbent articles 20, are provided at the end parts (radially outer ends) of the arms 346a. The suction pads 344a suction-hold the absorbent articles 20 by negative pressure. Suction surfaces, by which the suction pads 344a suction-hold the absorbent articles 20, have approximately the same shape and size as the absorbent articles 20. In other words, the suction pads 344a also have a longitudinal direction.

The arms 346a are cylindrical, and the arms 346a rotate about center axes of the cylindrical arms 346a, as do the arms 136 of the rotating device 130. The arms 346a rotate about the center axes of the cylindrical arms 346a in accordance with the rotation of the drum 342a. Specifically, when the arms 346a pass near the second conveying device 330a (when receiving the absorbent articles 20 from the second conveying device 330a), the longitudinal direction of the suction pads 344a provided at the end parts of the arms 346a approximately aligns with the circumferential direction of the cylindrical drum 342a. As a result of the arms 346a rotating about the center axes of the cylindrical arms 346a in accordance with the rotation of the drum 342a, a direction orthogonal to the longitudinal direction of the suction pads 134 provided at the end parts of the arms 136 is approximately aligned with the circumferential direction of the cylindrical drum 132 when the arms 346a pass near the third conveying device 350a (when delivering the absorbent articles 20 to the third conveying device 350a). Consequently, absorbent articles 20 conveyed in a state in which the longitudinal direction Da of the absorbent articles 20 is aligned with the second direction D2, which is the conveyance direction of the absorbent articles 20, are rotated by the third conveying device 350a so that the longitudinal direction Da of the absorbent articles 20 is orthogonal to the conveyance direction of the absorbent articles 20 (aligned with the first direction D1), and are delivered to the third conveying device 350a.

The arms 346a are able to move relative to the drum 342a along the circumferential direction of the drum 342a, and a distance between adjacent arms 346a can be adjusted. Due to this feature, the rotation and position adjustment device 340a shortens the distance between the absorbent articles 20 in the conveyance direction (second direction D2) in the third conveying device 350a not to "K1" (which is the case when the absorbent articles 20 are only rotated), but to "K2 (< K1)."

In the method for manufacturing absorbent articles 20 using such rotation and position adjustment devices 340a and 340b, it is possible to suppress equipment costs and improve the efficiency of manufacturing absorbent articles because the absorbent articles 20 are rotated and the distance between the absorbent articles 20 in the conveyance direction (second direction D2) is adjusted.

The third conveying device 350a, which receives the absorbent articles 20 from the rotation and position adjustment device 340a, is a conveyor that conveys the absorbent articles 20 while suctioning the absorbent articles 20 using negative pressure so that the absorbent articles 20 do not lift off of the conveying surface. The third conveying device 350a conveys rows of the absorbent articles 20 in the first conveying path R1. The third conveying device 350a conveys rows of the absorbent articles 20 in a direction orthogonal to the longitudinal direction Da of the absorbent articles 20, and supplies the absorbent articles 20 into the folding device 352a that, among the plurality of folding devices 352a for the absorbent articles 20, corresponds to the respective rows of the absorbent articles 20 (see FIG. 29, and step S6 of FIG. 25). The structure and function of the folding device 352a is the same as the structure and function of the folding device 190a described above, and therefore shall not be described here to avoid duplicate descriptions.

### (4-2) Modification B

In the above embodiment, an example was described of a method for manufacturing absorbent articles in which multiple rows of absorbent articles 20 conveyed on one conveying path are divided into two groups, and the absorbent articles 20 of one group are moved to another conveying path.

However, this example is not provided by way of limitation; in the method for manufacturing absorbent articles, M rows of absorbent articles 20 conveyed on one conveying path (where M is an integer of two or greater) may be divided into N groups (where N is an integer of 2 or greater and a number equal to or less than M, and preferably an integer of 2 or greater and a number less than M), and (N-1) groups of absorbent articles 20 may be moved to another conveying path. For example, in a method for manufacturing absorbent articles according to one example, it is possible for the M rows of absorbent articles 20 conveyed on the first conveying path R1 to be divided into groups respectively identified by "0," "1," ... "(N-1)," the identifying number being the remainder from dividing the row number by N, and the row number being determined by counting in the first direction D1 from the row of absorbent articles that is farthest upstream in the first direction D1 (the row number for the "0" group is divisible by N). In this manufacturing method, the absorbent articles 20 in the "(N-1)" group, which excludes the "1" group among the N groups, are moved by group to conveying paths other than the first conveying path R1 (a second conveying path to an Nth conveying path). The rows of absorbent articles conveyed on N conveying paths including the first conveying path R1 are conveyed in a direction orthogonal to the longitudinal direction of the absorbent articles, and are supplied into processing devices that, among a plurality of devices for processing the absorbent articles, correspond to respective rows of absorbent articles.

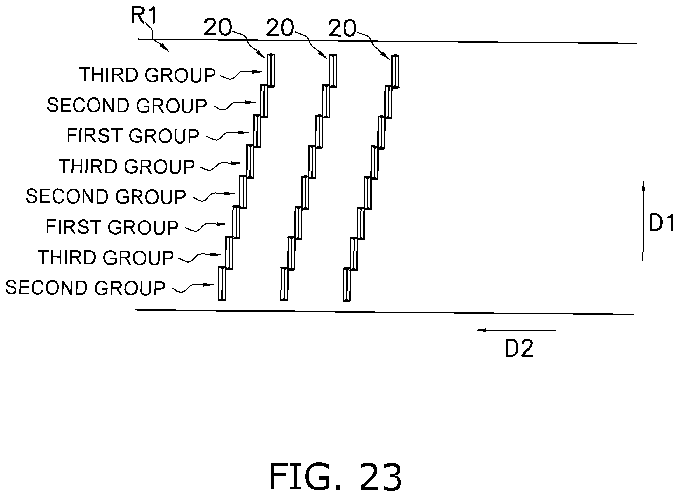

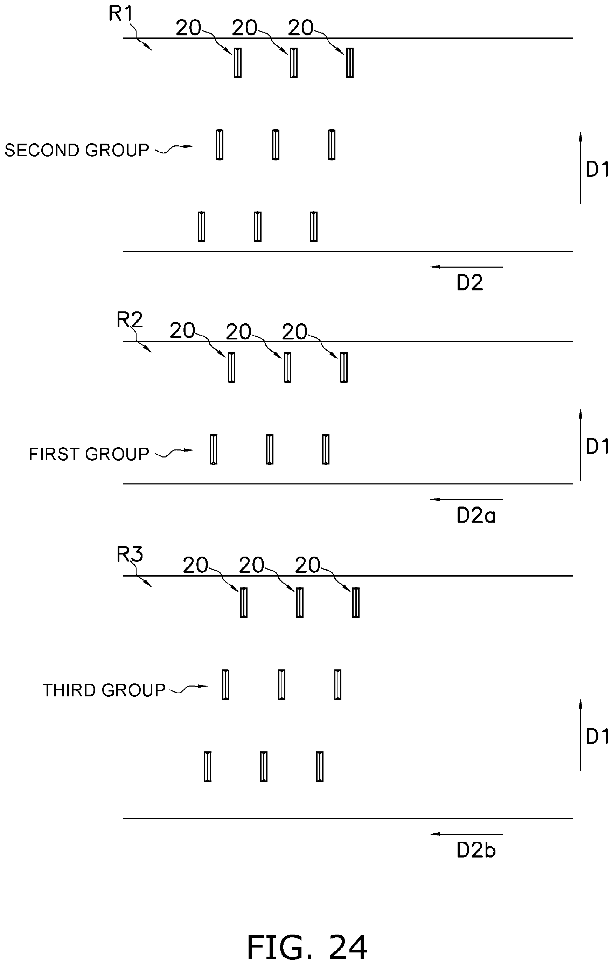

A specific example shall be described. For example, a case is envisioned in which eight rows of absorbent articles 20 conveyed on one conveying path (first conveying path R1) are divided into three groups, as shown in FIG. 23. Here, the eight rows of absorbent articles 20 are divided into a first group identified by "0" (third and sixth rows), a second group identified by "1" (first, fourth, and seventh rows), and a third group identified by "2" (second, fifth, and eighth rows), the identifying number being the remainder from dividing the row number by 3, and the row number being determined by counting along the first direction D1 from the row of absorbent articles that is farthest upstream in the first direction D1. Then, for example, as shown in FIG. 24, the absorbent articles 20 of the first group are moved from the first conveying path R1 to the second conveying path R2, and the absorbent articles 20 of the third group are moved from the first conveying path R1 to the third conveying path R3. The absorbent articles 20 of the second group continue to be conveyed on the first conveying path R1. Though not shown in the drawings, the rows of absorbent articles 20 conveyed on the first conveying path R1, the second conveying path R2, and the third conveying path R3 are conveyed in a direction orthogonal to the longitudinal direction of the absorbent articles 20, and are supplied into processing devices that, among the plurality of devices (e.g., the folding devices 190) for processing the absorbent articles 20, correspond to respective rows of absorbent articles.

In this method for manufacturing absorbent articles 20, absorbent articles aligned in multiple rows in the first direction D1 orthogonal to the second direction D2, which is the conveyance direction, are separated into N groups and then supplied into devices for processing the absorbent articles 20 corresponding to the respective rows of absorbent articles 20. Therefore, in this manufacturing method as well, space around each row of absorbent articles 20 will be readily ensured when the absorbent articles 20 are supplied into the processing devices, even if the distance between rows of absorbent articles 20 is not expanded. As a result, the incidence of events in which the absorbent articles 20 of adjacent rows impede other absorbent articles 20 from being supplied into the processing devices of the respective rows can be suppressed without expanding the conveyance width of the absorbent articles 20.

### (4-3) Modification C

In the above embodiment, during the manufacture of absorbent articles 20, the absorber cores 6c are arranged above the carrier sheets 6b (the absorber cores 6c are set above the carrier sheets 6b) and the absorbers 6 are arranged above the second divided sheets 4a (the absorbers 6 are set above the second divided sheets 4a). However, such an embodiment is not provided by way of limitation; in the steps of manufacturing the absorbent articles 20, the absorber cores 6c may be arranged above the cover sheets 6a and the absorbers 6 may be arranged above the second divided sheets 4a.

### (4-4) Modification D

In the above embodiment, the first divided sheets 2a, the second divided sheets 4a, the carrier sheets 6b, and the cover sheets 6a are formed by dividing raw material sheets (the first sheet 2, the second sheet 4, the third sheet 60a, and the fourth sheet 60b) into a plurality in a direction orthogonal to the conveyance direction of the raw material sheets. However, this example is not provided by way of limitation. For example, at least one among a plurality of web rolls to which a first sheet 2 having the same width as the first divided sheets 2a is wound, a plurality of web rolls to which a second sheet 4 having the same width as the second divided sheets 4a is wound, a plurality of web rolls to which a third sheet 60a having the same width as the carrier sheets 6b is wound, and/or a plurality of web rolls to which a fourth sheet 60b having the same width as the cover sheets 6a is wound may be used in the manufacture of the absorbent articles 20. In other words, the first divided sheets 2a, the second divided sheets 4a, the carrier sheets 6b, and/or the cover sheets 6a of the above embodiment need not be formed by dividing raw material sheets in the manufacturing device 100. From the beginning, sheets having the width in a direction orthogonal to the conveyance direction is substantially the same as the width in a direction orthogonal to the longitudinal direction Da of the absorbent articles 20 may be used instead of the first divided sheets 2a, the second divided sheets 4a, the carrier sheets 6b, and the cover sheets 6a.

Specifically, the method for manufacturing absorbent articles 20 of the present application may have, for example, a forming step, an arrangement step, a joining step, and a cutting-out step, such as the following. In the forming step, at least one among the conveyed first sheet 2, second sheet 4, third sheet 60a, and fourth sheet 60b (referred to as a first raw material sheet) is divided into a plurality in a direction orthogonal to the conveyance direction of the first raw material sheet, and a plurality of divided sheets are formed. In the arrangement step, a plurality of sheet members and absorber cores 6c are arranged. The plurality of sheet members include the divided sheets formed from the first raw material sheet, and raw material sheets (sheets not divided into a plurality in a direction orthogonal to the conveyance direction) other than the first raw material sheet. In the arrangement step, the absorber cores 6c are arranged between at least two sheet members. In the joining step, the absorber cores 6c and the sheet members opposing the absorber cores 6c are joined. In the cutting-out step, the sheet members joined to the absorber cores 6c are cut, and sheet pieces are cut out.

Finally, technical ideas that can be understood from the above embodiment and other examples (modifications) are additionally described below.

A method for manufacturing absorbent articles of a first aspect of the present invention comprises a conveyance step, a moving step, and a supplying step. In the conveyance step, multiple rows of absorbent articles aligned along a first direction are conveyed on a first conveyance path in a second direction orthogonal to the first direction. In the moving step, rows of absorbent articles that are either even-numbered or odd-numbered when counted along the first direction from the row of absorbent articles farthest upstream in the first direction are moved from a first conveying path to a second conveying path as rows of absorbent articles of divided groups while remaining in the same state of alignment in the first direction. The supplying step is performed after the rows of the absorbent articles of divided groups have been moved to the second conveying path. In the supplying step, in the first conveying path and the second conveying path, the rows of absorbent articles are conveyed in a direction orthogonal to a longitudinal direction of the absorbent articles, and are supplied into a processing device that corresponds to that row of absorbent articles among a plurality devices for processing the absorbent articles.

In the method for manufacturing absorbent articles according to the first aspect, absorbent articles aligned in multiple rows in a direction orthogonal to the conveyance direction are separated into odd-numbered rows and even-numbered rows, and are then supplied into absorbent-article-processing devices corresponding to the rows of absorbent articles. Therefore, even if a distance between the rows of absorbent articles is not expanded, space around the rows of absorbent articles is readily ensured when the absorbent articles are supplied into the processing devices. As a result, it is possible to suppress the incidence of events in which absorbent articles in adjacent rows impede absorbent articles from being supplied into the processing device of each row, without expanding the width at which the absorbent articles are conveyed.

A method for manufacturing absorbent articles according to a second aspect of the present invention is the method for manufacturing absorbent articles of the first aspect, wherein a distance in the first direction between the rows of absorbent articles conveyed on the first conveying path, before the rows of absorbent articles of divided groups are moved to the second conveying path, is less than a length of the absorbent articles in a longitudinal direction.

In the manufacturing method of the second aspect, the width of the first conveying path (width in a direction orthogonal to the conveyance direction) can be suppressed, and an installation area of a device for manufacturing absorbent articles can be suppressed.

A method for manufacturing absorbent articles according to a third aspect of the present invention is the method for manufacturing absorbent articles of the first aspect or the second aspect, further comprising a step in which the absorbent articles conveyed in a state in which the longitudinal direction of the absorbent articles is aligned with the conveyance direction of the absorbent articles are rotated so that the longitudinal direction of the absorbent articles is orthogonal to the conveyance direction of the absorbent articles.

In the manufacturing method of the third aspect, it is possible to suppress the width of the first conveying path (the width in a direction orthogonal to the conveyance direction) and to suppress the installation area of the manufacturing device compared to a case where the absorbent articles are constantly conveyed in a state in which the longitudinal direction of the absorbent articles is orthogonal to the conveyance direction of the absorbent articles.

A method for manufacturing absorbent articles according to a fourth aspect of the present invention is the method for manufacturing absorbent articles of the third aspect, wherein the step of rotating the absorbent articles is a step in which the absorbent articles conveyed on the first conveying path in a state in which the longitudinal direction of the absorbent articles and the second direction are aligned are rotated, before the rows of absorbent articles of divided groups are moved to the second conveying path, so that the longitudinal direction of the absorbent articles aligns with the first direction.

In the manufacturing method of the fourth aspect, there is no need to provide separate rotation devices for the absorbent articles conveyed on the first conveying path and the absorbent articles conveyed on the second conveying path. Therefore, in the manufacturing method of the fourth aspect, it is possible to suppress increase in the installation area of the manufacturing device and increase in the cost of the manufacturing device.

A method for manufacturing absorbent articles according to a fifth aspect of the present invention is the method for manufacturing absorbent articles of the third aspect or the fourth aspect, further comprising, after the step of rotating the absorbent articles, a step of reducing a distance between the absorbent articles in the conveyance direction of the absorbent articles, in each row of the absorbent articles.

In the manufacturing method of the fifth aspect, by bringing the absorbent articles closer together, it is possible to perform downstream processes (e.g., the steps of manufacturing packages containing the absorbent articles) at the same time on a plurality of absorbent articles. As a result, the manufacturing steps can be made more efficient, and the manufacturing device can be simplified.

A method for manufacturing absorbent articles according to a sixth aspect of the present invention is the method for manufacturing absorbent articles of the third aspect, wherein the step of rotating the absorbent articles is a step in which the absorbent articles conveyed on the first conveying path and the second conveying path in which the longitudinal direction of the absorbent articles and the second direction are aligned are rotated, after the rows of absorbent articles of divided groups are moved to the second conveying path, so that the longitudinal direction of the absorbent articles aligns with the first direction. In the step of rotating the absorbent articles, a distance between the absorbent articles in the conveyance direction of the absorbent articles is reduced in each row of the absorbent articles.

In the method for manufacturing absorbent articles according to the sixth aspect of the present invention, equipment costs can be suppressed and absorbent article manufacturing can be made more efficient because absorbent article rotation and adjustment of distance between absorbent articles in the conveyance direction can be performed at the same time.

A method for manufacturing absorbent articles according to a seventh aspect of the present invention is the method for manufacturing absorbent articles of any of the first through sixth aspects, wherein the first conveying path and the second conveying path are at least partially arranged at positions of different heights.

In the manufacturing method of the seventh aspect, space can be efficiently utilized in the height direction as well, and the area in which the manufacturing device 100 is installed is readily reduced.

A method for manufacturing absorbent articles according to another aspect of the present application comprises a conveying step, a moving step, and a supplying step. In the conveying step, absorbent articles aligned in M (an integer of 2 or greater) rows along a first direction are conveyed on a first conveying path in a second direction orthogonal to the first direction. In the moving step, row numbers when counted along the first direction from the row of absorbent articles farthest upstream in the first direction are grouped by row numbers having the same remainder when divided by N (an integer of 2 or greater and a number equal to or less than M). In the moving step, among the rows of absorbent articles in M rows of N groups, the rows of absorbent articles of (N-1) groups are each moved by group to different conveying paths while remaining in the same state of alignment in the first direction. The supplying step is performed after the rows of absorbent articles of (N-1) groups have been moved from the first conveying path to different conveying paths by group. In the supplying step, on N conveying paths including the first conveying path, rows of absorbent articles are conveyed in a direction orthogonal to the longitudinal direction of the absorbent articles, and are supplied into processing devices that, among a plurality of devices for processing the absorbent articles, correspond to respective rows of absorbent articles.

In this method for manufacturing absorbent articles, absorbent articles aligned in multiple rows in the first direction orthogonal to the second direction, which is the conveyance direction, are supplied into absorbent article processing devices corresponding to the rows of absorbent articles after being separated into N groups. Therefore, space around the rows of absorbent articles is readily ensured when the absorbent articles are supplied into the processing devices, even if the distance between the rows of absorbent articles is not expanded. As a result, it is possible to suppress the incidence of events in which absorbent articles in adjacent rows impede absorbent articles from being supplied into the processing device of each row, without expanding the width at which the absorbent articles are conveyed.

A method for manufacturing absorbent articles according to an eighth aspect of the present invention is used in the manufacture of absorbent articles that have an absorber and a sheet piece arranged adjacent to the absorber. The method for manufacturing absorbent articles comprises a forming step, an arrangement step, a joining step, and a cutting-out step. In the forming step, of a plurality of conveyed raw material sheets, at least a first raw material sheet is divided into a plurality in a direction orthogonal to a conveyance direction of the first raw material sheet, and a plurality of divided sheets are formed. In the arrangement step, a plurality of sheet members and absorbers are arranged. The plurality of sheet members include the divided sheets formed from the first raw material sheet and either raw material sheets other than the first raw material sheet or divided sheets formed by dividing the raw material sheets other than the first raw material sheet into a plurality in a direction orthogonal to the conveyance direction. In the arrangement step, the absorbers are arranged in between at least two of the sheet members. In the joining step, the absorbers and the sheet members opposing the absorbers are joined. In the cutting-out step, the sheet members joined to the absorbers are cut, and sheet pieces are cut out.

In this method for manufacturing absorbent articles, the first raw material sheet, which is a raw material sheet, is divided into a plurality in a direction orthogonal to the conveyance direction of the first raw material sheet, and a step of arranging the absorbers and the sheet members including the divided sheets is performed after the plurality of divided sheets have been formed. Therefore, it is possible to suppress the incidence of events in which inappropriate force is applied locally to the first raw material sheet when the first raw material sheet is cut and the sheet members constituting the absorbent articles become wrinkled or twisted, compare to a case where the first raw material sheet is divided into a plurality in a direction orthogonal to the conveyance direction of the first raw material sheet after the absorbers and the first raw material sheet have been arranged adjacent to each other.

### REFERENCE SIGNS LIST

- 2: First sheet (raw material sheet)
- 2a: First divided sheet (divided sheet)
- 2p: Sheet piece
- 4: Second sheet (raw material sheet)
- 4a: Second divided sheet (divided sheet)
- 4p: Sheet piece
- 6a: Cover sheet (divided sheet)
- 6b: Carrier sheet (divided sheet)
- 6: Absorber
- 20: Absorbent article
- 60a: Third sheet (raw material sheet)
- 60b: Fourth sheet (raw material sheet)

- 190a, 190b, 190c: Folding device (processing device)

- D1: First direction
- D2: Second direction
- Da: Longitudinal direction
- L: Length of absorbent article in longitudinal direction
- R1: First conveying path
- R2: Second conveying path

## Claims

1. A method for manufacturing absorbent articles, comprising:
a step in which absorbent articles aligned in multiple rows along a first direction are conveyed on a first conveying path in a second direction orthogonal to the first direction;
a step in which rows of absorbent articles that are either even-numbered or odd-numbered when counted along the first direction from the row of absorbent articles farthest upstream in the first direction are moved to a second conveying path different from the first conveying path as rows of absorbent articles of divided groups while remaining in the same state of alignment in the first direction; and
a step in which, after the rows of absorbent articles of divided groups have been moved to the second conveying path, the rows of absorbent articles are conveyed on the first conveying path and the second conveying path in a direction orthogonal to a longitudinal direction of the absorbent articles, and are supplied into a processing device that, among a plurality of devices for processing the absorbent articles, corresponds to the respective row of absorbent articles.

2. The method for manufacturing absorbent articles according to claim 1, wherein
a distance in the first direction between the rows of absorbent articles conveyed on the first conveying path, before the rows of absorbent articles of divided groups are moved to the second conveying path, is less than a length of the absorbent articles in a longitudinal direction.

3. The method for manufacturing absorbent articles according to claim 1 or 2,
further comprising a step in which the absorbent articles conveyed in a state in which the longitudinal direction of the absorbent articles is aligned with the conveyance direction of the absorbent articles are rotated so that the longitudinal direction of the absorbent articles is orthogonal to the conveyance direction of the absorbent articles.

4. The method for manufacturing absorbent articles according to claim 3, wherein
the step of rotating the absorbent articles is a step in which the absorbent articles conveyed on the first conveying path in a state in which the longitudinal direction of the absorbent articles and the second direction are aligned are rotated, before the rows of absorbent articles of divided groups are moved to the second conveying path, so that the longitudinal direction of the absorbent articles aligns with the first direction.

5. The method for manufacturing absorbent articles according to claim 3 or 4,
further comprising, after the step of rotating the absorbent articles, a step of reducing a distance between the absorbent articles in the conveyance direction of the absorbent articles, in each row of the absorbent articles.

6. The method for manufacturing absorbent articles according to claim 3, wherein
the step of rotating the absorbent articles is a step in which the absorbent articles conveyed on the first conveying path and the second conveying path in which the longitudinal direction of the absorbent articles and the second direction are aligned are rotated, after the rows of absorbent articles of divided groups are moved to the second conveying path, so that the longitudinal direction of the absorbent articles aligns with the first direction, and
in the step of rotating the absorbent articles, a distance between the absorbent articles in the conveyance direction of the absorbent articles is reduced in each row of the absorbent articles.

7. The method for manufacturing absorbent articles according to any one of claims 1 to 6, wherein
the first conveying path and the second conveying path are at least partially arranged at positions of different heights.

8. A method for manufacturing absorbent articles that have an absorber and a sheet piece arranged adjacent to the absorber, the method for manufacturing absorbent articles comprising:
a step in which, of a plurality of conveyed raw material sheets, at least a first raw material sheet is divided into a plurality in a direction orthogonal to a conveyance direction of the first raw material sheet, and a plurality of divided sheets are formed;
a step of arranging the absorbers and a plurality of sheet members that include the divided sheets formed from the first raw material sheet and either the raw material sheets other than the first raw material sheet or divided sheets formed by dividing the raw material sheets other than the first raw material sheet into a plurality in a direction orthogonal to the conveyance direction, the absorbers being arranged in between at least two of the sheet members;
a step of joining the absorbers and the sheet members opposing the absorbers; and
a step of cutting the sheet members to which the absorbers are joined to cut out the sheet pieces.
